(19) Europäisches Patentamt
European Patent Office
Office européen des brevets

(11) **EP 4 509 106 A1**

(12) **EUROPEAN PATENT APPLICATION**
published in accordance with Art. 153(4) EPC

(43) Date of publication:
**19.02.2025 Bulletin 2025/08**

(21) Application number: **23815686.3**

(22) Date of filing: **08.05.2023**

(51) International Patent Classification (IPC):
**A61F 13/53** (2006.01)  **A61F 13/534** (2006.01)
**A61F 13/537** (2006.01)

(52) Cooperative Patent Classification (CPC):
**A61F 13/53; A61F 13/534; A61F 13/537**

(86) International application number:
**PCT/JP2023/017275**

(87) International publication number:
**WO 2023/233925 (07.12.2023 Gazette 2023/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.05.2022 JP 2022087450**

(71) Applicant: **Sumitomo Seika Chemicals Co., Ltd.
Kako-gun, Hyogo 675-0145 (JP)**

(72) Inventor: **KITANAKA, Hironobu
Himeji-shi, Hyogo 672-8076 (JP)**

(74) Representative: **Vossius & Partner
Patentanwälte Rechtsanwälte mbB
Siebertstrasse 3
81675 München (DE)**

(54) **ABSORBENT BODY AND ABSORBENT ARTICLE**

(57)    The present invention relates to an absorber including a first water absorption layer, and a second water absorption layer laminated on the first water absorption layer, in which the first water absorption layer contains water-absorbent resin particles (I) having a water retention capacity of physiological saline of 32 to 80 g/g and a one-minute value of a lockup height of 1.6 cm or less, and the second water absorption layer contains water-absorbent resin particles (II) having a one-minute value of a lockup height of more than 1.6 cm.

*Fig.1*

EP 4 509 106 A1

**Description**

**Technical Field**

The present invention relates to an absorber and an absorbent article.

**Background Art**

From the viewpoint of increasing the absorption capacity and improving the dryness, an absorbent article in which a plurality of absorbers are laminated has been proposed. For example, Patent Literature 1 discloses an absorbent article including a top sheet having liquid permeability, a back sheet having liquid impermeability, and an absorber disposed between the top sheet and the back sheet, in which the absorber includes an upper layer absorbent core, a lower layer absorbent core which is provided on a skin non-contact side with respect to the upper layer absorbent core, an upper layer absorbent core penetrating portion that penetrates the upper layer absorbent core in a thickness direction, and a second sheet that is disposed to be in partial contact with an upper layer sheet constituting the absorbent article and disposed such that the upper layer sheet covers at least a part of a skin contact side of the upper layer absorbent core, in which the upper layer sheet and/or the second sheet is disposed to pass through an inside of the upper layer absorbent core penetrating portion.

**Citation List**

**Patent Literature**

[Patent Literature 1] Japanese Unexamined Patent Publication No. 2019-141309

**Summary of Invention**

**Technical Problem**

An object of the present invention is to provide an absorber that is a multilayer type absorber including a plurality of water absorption layers and that achieves both a high absorption rate and a small re-wet amount, and an absorbent article including the absorber.

**Solution to Problem**

[1] An absorber including: a first water absorption layer, and a second water absorption layer laminated on the first water absorption layer, in which the first water absorption layer contains water-absorbent resin particles (I) having a water retention capacity of physiological saline of 32 to 80 g/g and a one-minute value of a lockup height of 1.6 cm or less, the second water absorption layer contains water-absorbent resin particles (II) having a one-minute value of a lockup height of more than 1.6 cm, and the one-minute value of the lockup height is measured by the following procedures,

(1) 0.200 g of water-absorbent resin particles are disposed over an entire bottom surface in a cylinder having an inner diameter of 2.0 cm and a depth of 8.0 cm to form a particle layer, and a height of the particle layer is denoted by H0 [cm].
(2) 20 g of physiological saline is injected to the particle layer to swell the particle layer.
(3) The height of the particle layer is recorded as H1' one minute after a total amount of the physiological saline is injected, and a one-minute value [cm] of the lockup height is calculated from Expression: H1' - H0.

[2] The absorber according to [1], in which a content of the water-absorbent resin particles (I) contained in the first water absorption layer is in a range of 5% to 95% by mass with respect to a total amount of water-absorbent resin particles contained in the first water absorption layer, and a content of the water-absorbent resin particles (II) contained in the second water absorption layer is in a range of 60% to 100% by mass with respect to a total amount of water-absorbent resin particles contained in the second water absorption layer.
[3] The absorber according to [1] or [2], in which a basis weight of water-absorbent resin particles is in a range of 50 to 450 g/m$^2$.

[4] The absorber according to any one of [1] to [3], in which at least one of the first water absorption layer or the second water absorption layer contains a fibrous material.

[5] The absorber according to any one of [1] to [4], in which the absorber includes a plurality of layers of at least one of the first water absorption layer or the second water absorption layer.

[6] The absorber according to any one of [1] to [5], in which a water absorption rate of the water-absorbent resin particles (I) according to a Vortex method is more than 150 seconds.

[7] The absorber according to any one of [1] to [6], in which the water-absorbent resin particles (I) are coated-resin particles having a coating layer covering at least a part of a surface.

[8] An absorbent article including: the absorber according to any one of [1] to [7].

## Advantageous Effects of Invention

According to the present invention, it is possible to provide an absorber that is a multilayer type absorber including a plurality of water absorption layers and that achieves both a high absorption rate and a small re-wet amount.

## Brief Description of Drawings

Fig. 1 is a cross-sectional view showing an example of an absorber.
Fig. 2 is a cross-sectional view showing an example of the absorber.
Fig. 3 is a cross-sectional view showing an example of an absorbent article.

## Description of Embodiments

Hereinafter, several embodiments of the present invention will be described in detail. However, the present invention is not limited to the following embodiments.

In the present specification, "acryl" and "methacryl" collectively denote "(meth)acryl". "Acrylate" and "methacrylate" also similarly denote "(meth)acrylate". In the numerical value ranges described in a stepwise manner in the present specification, an upper limit or a lower limit of a numerical value range in a certain step can be optionally combined with an upper limit or a lower limit of a numerical value range in another step. In the numerical ranges described in the present specification, the upper limit or lower limit of the numerical ranges may be replaced with the values shown in the Examples. The materials exemplified in the present specification may be used alone or in combination of two or more kinds thereof. In a case where there are a plurality of substances corresponding to each component present in the composition, the content of each component in the composition means the total amount of the plurality of substances present in the composition unless otherwise specified. "Room temperature" means 25 $\pm$ 2°C. In addition to shape structures formed over the entire surface, the term "layer" also encompasses shape structures which are partially formed when observed in a plan view. "Physiological saline" denotes a 0.9 mass% sodium chloride aqueous solution.

[Absorber]

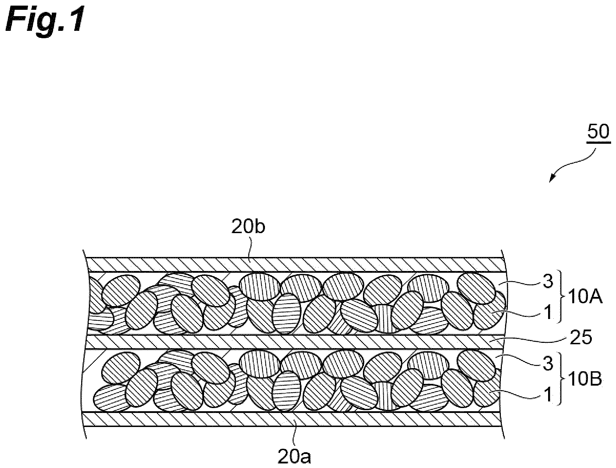

Fig. 1 is a cross-sectional view showing an example of an absorber. An absorber 50 shown in Fig. 1 includes sheet-shaped shape retaining members 20a and 20b and water absorption layers 10A and 10B. The water absorption layer 10B is laminated on the water absorption layer 10A via a separator 25. The water absorption layer 10B and the water absorption layer 10A may be laminated without sandwiching the separator 25 therebetween.

In the absorber 50, the water absorption layers 10A and 10B may each contain a plurality of water-absorbent resin particles 1 and may further contain a fibrous material 3. In the absorber 50, the water absorption layers 10A and 10B are surrounded by the shape retaining members 20a and 20b. The absorber 50 includes a separator 25 between the shape retaining members 20a and 20b.

The water absorption layer 10A is a first water absorption layer containing water-absorbent resin particles (I) having the water retention capacity of physiological saline of 32 to 80 g/g and the one-minute value of the lockup height of 1.6 cm or less.

In a case where the absorber 50 is used for an absorbent article such as a diaper, the water absorption layer 10A (first water absorption layer) may be disposed on a wearer side (that is, a side where the liquid to be absorbed enters) or may be disposed on a side opposite to the wearer side.

From the viewpoint of further reducing the re-wet amount, the lower limit of the water retention capacity of the physiological saline of the water-absorbent resin particles (I) may be 34 g/g or more, 36 g/g or more, 38 g/g or more, 40 g/g or more, or 42 g/g or more. From the viewpoint of more effectively suppressing occurrence of a gel blocking phenomenon, which is a factor that degrades the absorption rate, the upper limit of the water retention capacity of the physiological saline of the water-absorbent resin particles (I) may be 75 g/g or less, 70 g/g or less, 65 g/g or less, 60 g/g or less, 55 g/g or less, 50 g/g or less, or 45 g/g or less. From the viewpoint of achieving both a more improved absorption rate and a more improved re-wet amount, the water retention capacity of physiological saline of the water-absorbent resin particles (I) may be 32 to 70 g/g, 32 to 60 g/g, 34 to 50 g/g, or 36 to 46 g/g. In the present specification, "water retention capacity of physiological saline" is measured by a method described in examples below.

From the viewpoint of more effectively suppressing the occurrence of the gel blocking phenomenon and achieving both a more improved absorption rate and a more improved re-wet amount, the one-minute value of the lockup height of the water-absorbent resin particles (I) may be 1.4 cm or less, 1.2 cm or less, 1.0 cm or less, 0.8 cm or less, 0.6 cm or less, 0.4 cm or less, 0.2 cm or less, or 0.1 cm or less. The one-minute value of the lockup height of the water-absorbent resin particles (I) may be, for example, 0 cm or more or 0.1 cm or more.

In the present specification, "one-minute value of the lockup height" is measured by the following procedures.

(1) 0.200 g of water-absorbent resin particles are disposed over an entire bottom surface in a cylinder having an inner diameter of 2.0 cm and a depth of 8.0 cm to form a particle layer, and the height of the particle layer is denoted by H0 [cm].
(2) 20 g of physiological saline is injected to the particle layer to swell the particle layer.
(3) The height of the particle layer is recorded as H1' one minute after the total amount of the physiological saline is injected, and the one-minute value [cm] of the lockup height is calculated from Expression: H1' - H0. The details of the measurement procedures are as described in examples below.

The one-minute value of the lockup height can be adjusted within the above-mentioned range by, for example, a method of forming a coating layer which covers at least a part of the surface of the water-absorbent resin particles, a method of adjusting the shape, the particle diameter, the specific surface area, or the like of the water-absorbent resin particles, or a method of optionally combining these methods.

The water absorption rate of the water-absorbent resin particles (I) according to the Vortex method may be 100 seconds or more, 120 seconds or more, or 140 seconds or more, and from the viewpoint of suppressing the occurrence of the gel blocking phenomenon and achieving both an improved absorption rate and an improved re-wet amount, the water absorption rate thereof may be more than 150 seconds, 170 seconds or more, 190 seconds or more, 210 seconds or more, 230 seconds or more, 250 seconds or more, 270 seconds or more, or 290 seconds or more. The water absorption rate of the water-absorbent resin particles (I) according to the Vortex method may be, for example, 350 seconds or less, or 300 seconds or less. In the present specification, "water absorption rate according to the Vortex method" is measured by a method described in examples below.

The shape of the water-absorbent resin particles (I) is not particularly limited, and may be, for example, a true spherical shape, an amorphous shape, a substantially spherical shape, a crushed shape, a granular shape, or a shape in which primary particles having these shapes are aggregated (for example, a shape in which spherical or substantially spherical particles are aggregated).

The water-absorbent resin particles (I) may be used alone or in combination of a plurality of kinds of particles having different physical properties from each other in at least one of the water retention capacity of physiological saline or the one-minute value of the lockup height.

The water-absorbent resin particles (I) include polymer particles. The polymer particles may contain, for example, a crosslinked polymer having an ethylenically unsaturated monomer as a monomer unit. The polymer particles can be produced, for example, by a method including a step of polymerizing a monomer including an ethylenically unsaturated monomer. Examples of a polymerization method include a reverse phase suspension polymerization method, an aqueous solution polymerization method, a bulk polymerization method, and a precipitation polymerization method.

The ethylenically unsaturated monomer may be a water-soluble ethylenically unsaturated monomer. Examples of the water-soluble ethylenically unsaturated monomer include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth) acrylate, N,N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone or in combination of two or more kinds thereof.

In a case where the ethylenically unsaturated monomer has an acid group, the acid group may be neutralized using an alkaline neutralizing agent before being used in the polymerization reaction. The neutralization degree in the ethylenically unsaturated monomer due to the alkaline neutralizing agent may be, for example, 10% by mole to 100% by mole, 50% by mole to 90% by mole, or 60% by mole to 80% by mole of the acid groups in the ethylenically unsaturated monomer. Examples of the alkaline neutralizing agent include alkali metal salts such as sodium hydroxide, sodium carbonate, sodium hydrogen carbonate, potassium hydroxide, and potassium carbonate, and ammonia. The alkaline neutralizing agent may be used alone or in combination of two or more kinds thereof. The alkaline neutralizing agent may be used in the form of an aqueous solution to simplify the neutralization operation.

The polymer particles may further have a monomer unit derived from a monomer other than the above-mentioned ethylenically unsaturated monomer. The proportion of the monomer unit derived from an ethylenically unsaturated monomer may be 70% to 100% by mole with respect to the total amount of the monomer units constituting the polymer particles. The proportion of the monomer unit derived from the ethylenically unsaturated monomer may be 80% to 100% by mole, 85% to 100% by mole, 90% to 100% by mole, or 95% to 100% by mole with respect to the total amount of the monomer units constituting the polymer particles. The proportion of the monomer unit derived from (meth) acrylic acid and a salt thereof may be 70% to 100% by mole with respect to the total amount of the monomer units constituting the crosslinked polymer in the polymer particles. The proportion of the monomer unit derived from (meth) acrylic acid and a salt thereof may be 80% to 100% by mole, 85% to 100% by mole, 90% to 100% by mole, or 95% to 100% by mole with respect to the total amount of the monomer units constituting the crosslinked polymer in the polymer particles.

The crosslinked polymer constituting the polymer particles may be crosslinked with an internal crosslinking agent. The internal crosslinking agent is usually added to the reaction solution during the polymerization reaction. In a case of using the internal crosslinking agent, the water-absorbent characteristics of the water-absorbent resin particles (I) (the water retention capacity of physiological saline, the water absorption rate according to the Vortex method, and the like) are easily controlled.

The polymer particles may be crosslinked with a surface crosslinking agent. The polymer in the vicinity of the surface of the polymer particles is mainly crosslinked by the surface crosslinking agent. In a case of using the surface crosslinking agent, the water-absorbent characteristics of the water-absorbent resin particles (the water retention capacity of physiological saline, the water absorption rate according to the Vortex method, and the like) are easily controlled.

The polymer particles may be substantially formed of only the crosslinked polymer, but may further contain, for example, various additional components selected from a gel stabilizer, a metal chelating agent, a flowability improver (lubricant), and the like. The additional components can be disposed inside the polymer particles, on the surface of the polymer particles, or both thereof. The additional component may be a flowability improver (lubricant). The flowability improver may contain inorganic particles. Examples of the inorganic particles include silica particles such as amorphous silica. In a case where the water-absorbent resin particles contain inorganic particles, the content of the inorganic particles may be 0.05 parts by mass or more, 0.1 parts by mass or more, 0.2 parts by mass or more, 0.3 parts by mass or more, or 0.4 parts by mass or more, and may be 5.0 parts by mass or less, 3.0 parts by mass or less, 1.0 parts by mass or less, 0.7 parts by mass or less, or 0.5 parts by mass or less with respect to 100 parts of the total mass of the polymer particles.

The water-absorbent resin particles (I) may be classified using a sieve or may not be classified. The water-absorbent resin particles may be water-absorbent resin particles having a particle diameter of less than 850 $\mu$m. The expression "particle diameter of less than 850 $\mu$m" denotes that the particles can pass through a JIS standard sieve having an opening of 850 $\mu$m. The water-absorbent resin particles (I) may be water-absorbent resin particles having a particle diameter of 250 $\mu$m or more. The expression "particle diameter of 250 $\mu$m or more" denotes that the particles do not pass through a JIS standard sieve having an opening of 250 $\mu$m. The water-absorbent resin particles may be water-absorbent resin particles having a particle diameter of 250 $\mu$m or more and less than 850 $\mu$m (water-absorbent resin particles that pass through a JIS standard sieve having an opening of 850 $\mu$m and do not pass through a JIS standard sieve having an opening of 250 $\mu$m).

The water-absorbent resin particles (I) may be substantially formed of only polymer particles, but may be, for example, coated-resin particles having polymer particles and a coating layer that covers at least a part of the surface of the polymer particles. By forming a coating layer which covers at least a part of the surface of the polymer particles, the water-absorbent resin particles in which the one-minute value of the lockup height is low are more likely to be obtained.

The coating layer may contain a homopolymer, a copolymer, or a mixture thereof, which contains a substituted or unsubstituted alkene as a monomer unit, and a homopolymer, a copolymer, or a mixture thereof, which contains alkylene oxide as a monomer unit. The copolymer containing an unsubstituted alkene as a constitutional unit may be a copolymer containing only two or more kinds of unsubstituted alkenes as a monomer unit, a copolymer containing one or two or more kinds of unsubstituted alkenes and a monomer other than the unsubstituted alkenes as a monomer unit, or a copolymer containing one kind of unsubstituted alkene and a monomer other than the unsubstituted alkene as a monomer unit.

Examples of the unsubstituted alkene constituting the copolymer include ethylene, propylene, and butene. The unsubstituted alkene may be ethylene and/or propylene, or may be ethylene.

The monomer other than the unsubstituted alkene, which constitutes the copolymer, may be a water-soluble ethylenically unsaturated monomer. The water-soluble ethylenically unsaturated monomer can be the same as the compound exemplified as the water-soluble ethylenically unsaturated monomer constituting the polymer particles, and may be (meth)acrylic acid and/or a salt thereof.

The copolymer containing an unsubstituted alkene as a monomer unit may be a copolymer containing ethylene and a water-soluble ethylenically unsaturated monomer as a monomer unit, a copolymer containing ethylene, (meth)acrylic acid, and/or a salt thereof as a monomer unit, a copolymer containing ethylene and an acrylic acid salt as a monomer unit, a copolymer containing ethylene and sodium acrylate, potassium acrylate, or ammonium acrylate as a monomer unit (an ethylene-sodium acrylate copolymer, an ethylene-potassium acrylate copolymer, or an ethylene-ammonium acrylate), or an ethylene-sodium acrylate copolymer.

The polymer containing alkylene oxide as a monomer unit may be a polyalkylene oxide (homopolymer) containing only one kind of alkylene oxide as a monomer unit, polyalkylene oxide (copolymer) containing two or more kinds of alkylene oxides as a monomer unit, or a copolymer containing one or two or more kinds of alkylene oxides and a monomer other than alkylene oxide as a monomer unit. The polymer containing alkylene oxide as a monomer unit may be polyalkylene oxide containing only one kind of alkylene oxide as a monomer unit.

Examples of the alkylene oxide include ethylene oxide and propylene oxide. The alkylene oxide may be particularly ethylene oxide.

The polymer containing alkylene oxide as a monomer unit may be a homopolymer (polyethylene glycol) containing ethylene oxide as a monomer unit, a copolymer containing ethylene oxide and propylene oxide as monomer units, or a combination thereof, and may be polyethylene glycol.

The total content of the coating layer may be, for example, 1 to 40 parts by mass with respect to 100 parts by mass of the polymer particles on which the coating layer is formed.

The coating layer can be formed on at least a part of the surface, for example, by bringing the polymer particles into contact with a coating material. The coating material is, for example, a component capable of forming the above-mentioned coating layer or a material for forming the component.

The coating layer can be formed, for example, by (1) a method using an eggplant flask, (2) a method using a sprayer, or (3) a method using various granulators.

(1) Method Using Eggplant Flask

In the method using an eggplant flask, first, a coating material is injected into the eggplant flask, and then polymer particles are injected into the eggplant flask. The eggplant flask is attached to an evaporator and heated while rotating, and the liquid medium contained in the coating material is distilled off under reduced pressure conditions. In this manner, coated-resin particles in which the surface of the polymer particles is coated with the coating layer are obtained.

(2) Method Using Sprayer

In the method using a sprayer, first, the polymer particles are added to a separable flask provided with a stirrer blade, and the mixture is stirred. The polymer particles wound up by being stirred with the stirrer blade are sprayed with a coating material. It is possible to spray the coating material using, for example, a two-fluid type nozzle. From the viewpoint that uniform coating can be expected, it is desirable for the coating material to be sprayed in a mist form using an air stream of an inert gas such as nitrogen. Thereafter, the contents of the separable flask are taken out, heated by a hot air dryer, and then cooled to room temperature to obtain coated-resin particles.

(3) Method Using Various Granulators

Examples of granulators used for producing coated-resin particles include a rolling granulator, a stirring granulator, and a fluidized bed granulator.

In a case where a rolling granulator is used, an inclined shallow circular container attached to the rolling granulator rotates in advance, and polymer particles are supplied to the circular container and an appropriate amount of the coating material is added. In this manner, the rolling particles are partially aggregated and a coating layer is formed on the surface thereof by the solvent or the dispersion medium contained in the coating material. Further, the adding step of adding the polymer particles and the coating material can be performed a plurality of times as necessary.

In a case where a stirring granulator is used, the polymer particles are injected into a mixer attached to the stirring granulator and stirred to be mixed, and the coating material is added thereto. In this manner, the particles during stirring are partially aggregated and a coating layer is formed on the surface thereof by the liquid medium contained in the coating material. The adding step of adding the polymer particles and the coating material can be performed a plurality of times as necessary. Further, excessive aggregation of the polymer particles can be suppressed by controlling the shearing force of the mixer.

In a case where a fluidized bed granulator is used, first, the polymer particles are injected into a container, which is attached to the fluidized bed granulator and in which hot air can be sent out from a lower portion, and the polymer particles are preliminarily fluidized. Thereafter, in a case where the coating material is sprayed from a nozzle provided in the container, the polymer particles during stirring are partially aggregated and a coating layer is formed on the surface thereof by the liquid medium contained in the coating material. It is possible to perform the spraying of the coating material a plurality of times as necessary. The excessive aggregation of the polymer particles can be suppressed by adjusting the spraying amount and/or the spraying frequency of the coating material. For example, a fluidized bed granulator FBD/SG (manufactured by Yenchen Machinery Co., Ltd.) can be used as the fluidized bed granulator.

From the viewpoint that the balance between the absorption rate and the re-wet amount is more improved, the content of the water-absorbent resin particles (I) contained in the first water absorption layer may be 5% to 95% by mass, 5% to 75% by mass, 10% to 55% by mass, 10% to 45% by mass, 15% to 35% by mass, or 15% to 25% by mass with respect to the total amount of the water-absorbent resin particles contained in the first water absorption layer.

The first water absorption layer may further contain water-absorbent resin particles other than the water-absorbent resin particles (I). The water-absorbent resin particles other than the water-absorbent resin particles (I) are water-absorbent resin particles in which the water retention capacity of physiological saline is less than 32 g/g or more than 80 g/g, or the one-minute value of the locked height is more than 1.6 cm.

The first water absorption layer may further contain water-absorbent resin particles (II) in which the one-minute value of the locked height is more than 1.6 cm.

The one-minute value of the lockup height of the water-absorbent resin particles (II) may be 1.7 cm or more, 1.8 cm or more, 1.9 cm or more, 2.0 cm or more, 2.2 cm or more, 2.4 cm or more, or 2.6 cm or more, and may be 4.5 cm or less, 4.0 cm or less, 3.5 cm or less, 3.0 cm or less, 2.8 cm or less, 2.6 cm or less, 2.4 cm or less, 2.2 cm or less, 2.0 cm or less, or 1.8 cm or less. The one-minute value of the lockup height of the water-absorbent resin particles (II) may be, for example, more than 1.6 cm and 4.5 cm or less, more than 1.6 cm and 4.0 cm or less, more than 1.6 cm and 3.5 cm or less, more than 1.6 cm and 3.0 cm or less, more than 1.6 cm and 2.8 cm or less, more than 1.6 cm and 2.6 cm or less, or more than 1.6 cm and 2.4 cm or less.

The water retention capacity of physiological saline of the water-absorbent resin particles (II) may be, for example, 32 to 80 g/g. The lower limit of the water retention capacity of physiological saline of the water-absorbent resin particles (II) may be 34 g/g or more, 36 g/g or more, or 38 g/g or more, and from the viewpoint that the effect of achieving both a high absorption rate and a small re-wet amount is further improved, the lower limit thereof is preferably 40 g/g or more or 42 g/g or more. The upper limit of the water retention capacity of the physiological saline of the water-absorbent resin particles (II) may be, for example, 75 g/g or less, 70 g/g or less, 65 g/g or less, 60 g/g or less, 55 g/g or less, 50 g/g or less, or 45 g/g or less.

The water retention capacity of physiological saline of the water-absorbent resin particles (II) may be less than 32 g/g or more than 80 g/g. The water retention capacity of physiological saline of the water-absorbent resin particles (II) may be, for example, 20 g/g or more and less than 32 g/g, 25 g/g or more and less than 32 g/g, or 28 g/g or more and less than 32 g/g, and may be more than 80 g/g and 90 g/g or less or more than 80 g/g and 85 g/g or less.

From the viewpoint that the effect of achieving both a high absorption rate and a small re-wet amount is further improved, the water retention capacity of physiological saline of the water-absorbent resin particles (II) may be more than the water retention capacity of physiological saline of the water-absorbent resin particles (I). A difference Xb - Xa between the water retention capacity Xb (g/g) of the physiological saline of the water-absorbent resin particles (II) and the water retention capacity Xa (g/g) of the physiological saline of the water-absorbent resin particles (I) may be, for example, 1 or more, 1 to 15, 1 to 12, 1 to 10, 1 to 5, or 1 to 3.

The water absorption rate of the water-absorbent resin particles (II) according to the Vortex method may be, for example, 150 seconds or less, 120 seconds or less, 90 seconds or less, 80 seconds or less, 70 seconds or less, 60 seconds or less, 50 seconds or less, or 40 seconds or less. The water absorption rate of the water-absorbent resin particles (II) according to the Vortex method may be, for example, 15 seconds or more, 20 seconds or more, 25 seconds or more, 30 seconds or more, or 35 seconds or more.

The first water absorption layer may further contain the water-absorbent resin particles (III) in which the water retention capacity of physiological saline is less than 32 g/g or more than 80 g/g and the one-minute value of the lockup height is 1.6 cm or less in place of or in addition to the water-absorbent resin particles (II).

The water retention capacity of physiological saline of the water-absorbent resin particles (III) may be, for example, 20 g/g or more and less than 32 g/g, 25 g/g or more and less than 32 g/g, or 28 g/g or more and less than 32 g/g, and may be more than 80 g/g and 90 g/g or less or more than 80 g/g and 85 g/g or less. The one-minute value of the lockup height of the water-absorbent resin particles (III) may be, for example, in the above-mentioned numerical ranges described in the section of the water-absorbent resin particles (I).

The water absorption rate of the water-absorbent resin particles (III) according to the Vortex method may be, for example, 150 seconds or less, 120 seconds or less, 90 seconds or less, 80 seconds or less, 70 seconds or less, 60 seconds or less, 50 seconds or less, or 40 seconds or less. The water absorption rate of the water-absorbent resin particles (III) according to the vortex method may be, for example, 15 seconds or more, 20 seconds or more, 25 seconds or more, 30 seconds or more, or 35 seconds or more.

The total content of the water-absorbent resin particles other than the water-absorbent resin particles (I) in the first water absorption layer (the mass of the water-absorbent resin particles (II) and the total mass of the water-absorbent resin particles (III)) may be 5% by mass or more, 10% by mass or more, 20% by mass or more, 30% by mass or more, 40% by mass or more, 50% by mass or more, 60% by mass or more, 70% by mass or more, or 75% by mass or more with respect to the total amount of the first water-absorbent resin particles contained in the first water absorption layer. The content of the water-absorbent resin particles (III) contained in the first water absorption layer may be 95% by mass or less, 85% by mass or less, 75% by mass or less, 65% by mass or less, 55% by mass or less, 45% by mass or less, 35% by mass or less, or 25% by mass or less, with respect to the total amount of the water-absorbent resin particles contained in the first water absorption layer. The content of the water-absorbent resin particles (II) contained in the first water absorption layer may be the above-mentioned total content.

The water-absorbent resin particles (II) and (III) can be obtained by the same method as the method for the water-absorbent resin particles (I) except that the water retention capacity of physiological saline is adjusted to less than 32 g/g or more than 80 g/g or the one-minute value of the lockup height is adjusted to more than 1.6 cm.

The first water absorption layer may contain the water-absorbent resin particles (I) and the water-absorbent resin particles (II).

The total mass of the water-absorbent resin particles contained in the first water absorption layer may be, for example, 5% by mass or more, 10% by mass or more, 15% by mass or more, 20% by mass or more, 25% by mass or more, 30% by mass or more, 35% by mass or more, 40% by mass or more, 45% by mass or more, 50% by mass or more, or 55% by mass or more with respect to the total mass of the first water absorption layer, and may be 100% by mass or less, 95% by mass or less, 90% by mass or less, 85% by mass or less, 80% by mass or less, 75% by mass or less, 70% by mass or less, or 65% by mass or less. The mass of the water-absorbent resin particles (I) and the mass of the water-absorbent resin particles (II) may be in the ranges of the mass described as the total mass of the water-absorbent resin particles contained in the first water absorption layer.

The first water absorption layer may or may not contain a fibrous material. In the present specification, the fibrous material has an amorphous shape as a whole, and does not include fibrous materials molded in the sheet shape, such as non-woven fabrics. Examples of the fibrous material include crushed pulp (crushed pulp), finely pulverized wood pulp, cotton, cotton linter, rayon, cellulosic fibers such as cellulose acetate, synthetic fibers such as polyamide, polyester, and polyolefin, and a mixture of these fibers. The fibrous material may be used alone or in combination of two or more kinds thereof. As the fibrous material, hydrophilic fibers can be used.

In a case where the first water absorption layer contains the fibrous material, the content of the fibrous material may be, for example, 5% by mass or more, 10% by mass or more, 15% by mass or more, 20% by mass or more, 25% by mass or more, 30% by mass or more, or 35% by mass or more with respect to the total mass of the first water-absorbent resin particles and the total mass of the fibrous material in the first water absorption layer. The content of the fibrous material may be 100% by mass or less, 95% by mass or less, 90% by mass or less, 85% by mass or less, 80% by mass or less, 75% by mass or less, 70% by mass or less, 65% by mass or less, 60% by mass or less, 55% by mass or less, 50% by mass or less, or 45% by mass or less with respect to the total mass of the water-absorbent resin particles and the total mass of the fibrous material in the first water absorption layer.

The water absorption layer 10B is a second water absorption layer containing the above-mentioned water-absorbent resin particles (II). In a case where the absorber 50 is used in an absorbent article such as a diaper, the second water absorption layer may be disposed on the wearer side (that is, a side where the liquid to be absorbed enters) or may be disposed on a side opposite to the wearer side.

The content of the water-absorbent resin particles (II) contained in the second water absorption layer may be 60% to 100% by mass with respect to the total amount of the water-absorbent resin particles contained in the second water absorption layer. The lower limit of the content of the water-absorbent resin particles (II) contained in the second water absorption layer may be 65% by mass or more, 70% by mass or more, 75% by mass or more, 80% by mass or more, 85% by mass or more, 90% by mass or more, or 95% by mass or more with respect to the total amount of the water-absorbent resin particles contained in the second water absorption layer. The upper limit of the content of the water-absorbent resin particles (II) contained in the second water absorption layer is 100% by mass or less, and may be, for example, 95% by mass or less, 90% by mass or less, or 85% by mass or less, with respect to the total amount of the water-absorbent resin particles contained in the second water absorption layer.

The second water absorption layer may or may not further contain water-absorbent resin particles other than the water-absorbent resin particles (II). The water-absorbent resin particles other than the water-absorbent resin particles (II) are water-absorbent resin particles in which the one-minute value of the lockup height is 1.6 cm or less.

In a case where the second water absorption layer further contains water-absorbent resin particles other than the water-absorbent resin particles (II), the second water absorption layer may further contain, for example, the above-mentioned water-absorbent resin particles (I) and/or the above-mentioned water-absorbent resin particles (III). In a case where the second water absorption layer further contains water-absorbent resin particles other than the water-absorbent resin particles (II), the compositions (combination of water-absorbent resin particles and the like) of the first water absorption layer and the second water absorption layer may be the same as or different from each other.

The content of the water-absorbent resin particles other than the water-absorbent resin particles (II) may be less than 35% by mass, 30% by mass or less, less than 25% by mass, 20% by mass or less, 15% by mass or less, 10% by mass or less, 5% by mass or less, or 1% by mass or less with respect to the total amount of the water-absorbent resin particles contained in the second water absorption layer. In a case where the second water absorption layer contains water-absorbent resin particles other than the water-absorbent resin particles (II), the lower limit of the content of the water-absorbent resin particles other than the water-absorbent resin particles (II) contained in the second water absorption layer may be 1% by mass or more, 5% by mass or more, 10% by mass or more, 15% by mass or more, or 20% by mass or more with respect to the total amount of the water-absorbent resin particles contained in the second water absorption layer. The content of the water-absorbent resin particles (I) in the second water absorption layer may be "content of the water-absorbent resin particles other than the water-absorbent resin particles (II)" described above.

The total mass of the water-absorbent resin particles contained in the second water absorption layer may be, for example, 5% by mass or more, 10% by mass or more, 15% by mass or more, 20% by mass or more, 25% by mass or more, 30% by mass or more, 35% by mass or more, 40% by mass or more, 45% by mass or more, 50% by mass or more, or 55% by mass or more, and may be 100% by mass or less, 95% by mass or less, 90% by mass or less, 85% by mass or less, 80% by mass or less, 75% by mass or less, 70% by mass or less, or 65% by mass or less with respect to the total mass of the second water absorption layer.

The second water absorption layer may or may not contain a fibrous material. The fibrous material that can be contained in the second water absorption layer may be the same as the fibrous material described in the section of the first water absorption layer.

In a case where the second water absorption layer contains a fibrous material, the content of the fibrous material may be, for example, 5% by mass or more, 10% by mass or more, 15% by mass or more, 20% by mass or more, 25% by mass or more, 30% by mass or more, or 35% by mass or more with respect to the total mass of the water-absorbent resin particles and the total mass of the fibrous material in the second water absorption layer. The content of the fibrous material may be 100% by mass or less, 95% by mass or less, 90% by mass or less, 85% by mass or less, 80% by mass or less, 75% by mass or less, 70% by mass or less, 65% by mass or less, 60% by mass or less, 55% by mass or less, 50% by mass or less, or 45% by mass or less with respect to the total mass of the water-absorbent resin particles and the total mass of the fibrous material in the second water absorption layer.

In the absorber 50, at least one of the first water absorption layer or the second water absorption layer may contain a fibrous material, or both the first water absorption layer and the second water absorption layer may contain a fibrous material. In the absorber 50, both the first water absorption layer and the second water absorption layer may not contain a fibrous material.

From the viewpoint that the effect of achieving both a high absorption rate and a small re-wet amount is further improved, a ratio (S1/S2 × 100) of an area S1 of a region (region 1) where the first water absorption layer is provided and overlaps with a region (region 2) to an area S2 of the region (region 2) where the second water absorption layer is provided in plan view may be 20% to 100%, 30% to 100%, 40% to 100%, 50% to 100%, 60% to 100%, 70% to 100%, 80% to 100%, 85% to 100%, 90% to 100%, or 95% to 100%.

In the absorber 50, the shape retaining members 20a and 20b are sheets provided to surround the water-absorbent resin particles 1 and the fibrous material 3 which is optionally added. The shape retaining member 20b is disposed on a side of the water absorption layer 10A opposite to the water absorption layer 10B side in a state of being in contact with the water absorption layer 10A. The shape retaining member 20a is disposed on a side opposite to the water absorption layer 10A side in a state of being in contact with the water absorption layer 10B. That is, the water absorption layers 10A and 10B are disposed between the shape retaining members 20a and 20b. The water absorption layers 10A and 10B are not necessarily completely surrounded by the shape retaining members 20a and 20b, and for example, an end portion of the water absorption layer 10A and/or the water absorption layer 10B may be exposed to the outside. The shape retaining members 20a and 20b may be two separate sheets or one folded sheet. The shape retaining members 20a and 20b may be non-woven fabrics such as airlaid non-woven fabrics, or may be tissue paper.

An adhesive layer containing an adhesive may be provided between the shape retaining member 20b and the water absorption layer 10A or between the shape retaining member 20a and the water absorption layer 10B, as necessary. Examples of the adhesive include a hot melt adhesive. Examples of the hot melt adhesives include a mixture of a base polymer, such as an ethylene-vinyl acetate copolymer, a styreneisoprene-styrene block copolymer, a styrene-butadiene-styrene block copolymer, a styrene-ethylene-butylene-styrene block copolymer, a styrene-ethylene-propylene-styrene block copolymer, or amorphous polypropylene, with a tackifier, a plasticizer, an antioxidant, or the like.

The separator 25 is provided to divide the water absorption layers 10A and 10B into two regions. The separator 25 may be a non-woven fabric such as an airlaid non-woven fabric, an air-through non-woven fabric, a spunbond non-woven fabric, or a spunlace non-woven fabric, or may be tissue paper. The separator 25 may be, for example, one air-through non-woven fabric, a laminate of two sheets of tissue paper, or a laminate of tissue paper and an airlaid non-woven fabric.

Fig. 1 is an example, and the configuration of the absorber is not limited thereto. For example, the absorber may further include one or a plurality of water absorption layers (other water absorption layers) in addition to the water absorption layers 10A and 10B. The total number of the water absorption layers in the absorber may be, for example, 2 to 5, 2 to 4, or 2 to 3.

The aspects described in the section of the first water absorption layer or the second water absorption layer can be applied to the specific aspects of the other water absorption layers, such as the kind or content of the water-absorbent resin particles contained in the other water absorption layers, or the kind or content of the fibrous material contained in the other water absorption layers.

The other water absorption layers may be a first water absorption layer containing the water-absorbent resin particles (I) or a second water absorption layer containing the water-absorbent resin particles (II), or may be a water absorption layer (third water absorption layer) other than the first water absorption layer and the second water absorption layer. The third water absorption layer may be, for example, a water absorption layer containing the above-mentioned water-absorbent resin particles (III) without containing both the water-absorbent resin particles (I) and the water-absorbent resin particles (II).

The absorber may include a plurality of layers of at least one of the first water absorption layer or the second water absorption layer. The absorber may include, for example, one first water absorption layer and two second water absorption layers.

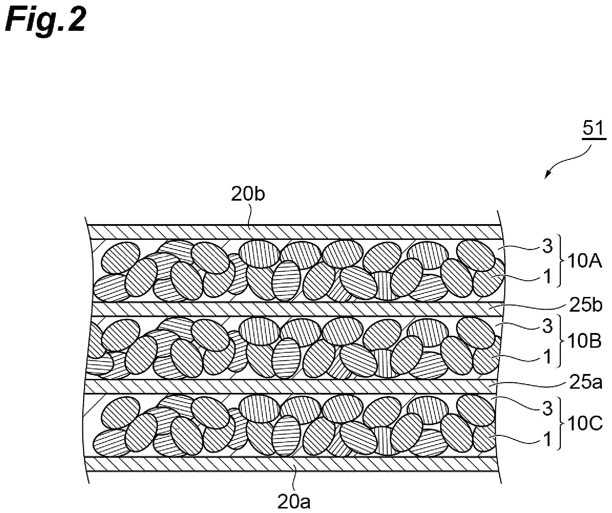

Fig. 2 is a cross-sectional view showing another example of the absorber. An absorber 51 shown in Fig. 2 includes the sheet-shaped shape retaining members 20a and 20b, and the water absorption layers 10A, 10B, and 10C. The water absorption layer 10A, the water absorption layer 10B, and the water absorption layer 10C are laminated in this order. The absorber 51 includes the separators 25a and 25b between the shape retaining members 20a and 20b. The separator 25a is provided to divide the water absorption layers 10A and 10B into two regions. The separator 25b is provided to divide the water absorption layers 10B and 10C into two regions.

Among the water absorption layers 10A, 10B, and 10C, at least one layer is the first water absorption layer containing the water-absorbent resin particles (I), and at least one layer is the second water absorption layer containing the water-absorbent resin particles (II). Any of the water absorption layers 10A, 10B, and 10C is not necessarily the third water absorption layer, and one layer of the water absorption layers 10A, 10B, and 10C may be the third water absorption layer.

The absorber 51 may be, for example, an absorber in which the water absorption layer 10A is the first water absorption layer and both the water absorption layers 10B and 10C are the second water absorption layers, an absorber in which the water absorption layers 10A and 10B are both the first water absorption layers and the water absorption layer 10C is the second water absorption layer, an absorber in which both the water absorption layers 10A and 10C are the first water absorption layers and the water absorption layer 10B is the second water absorption layer, or an absorber in which the water absorption layer 10B is the first water absorption layer and both the water absorption layers 10A and 10C are the second water absorption layers.

In a case where the absorber 51 is used in an absorbent article such as a diaper, the first water absorption layer may be disposed on the wearer side, the second water absorption layer may be disposed on the wearer side, or the third water absorption layer may be disposed on the wearer side.

From the viewpoint that the effect of achieving both a high absorption rate and a small re-wet amount is further improved, the basis weight of the water-absorbent resin particles in the absorber 50 (or the absorber 51) may be 75 $g/m^2$ or more, 100 $g/m^2$ or more, 125 $g/m^2$ or more, 150 $g/m^2$ or more, 175 $g/m^2$ or more, 200 $g/m^2$ or more, 225 $g/m^2$ or more, 250 $g/m^2$ or more, 275 $g/m^2$ or more, or 300 $g/m^2$ or more, and 450 $g/m^2$ or less, 425 $g/m^2$ or less, 400 $g/m^2$ or less, 375 $g/m^2$ or less, 350 $g/m^2$ or less, 325 $g/m^2$ or less, 300 $g/m^2$ or less, 275 $g/m^2$ or less, or 260 $g/m^2$ or more. From the viewpoint that the effect of achieving both a high absorption rate and a small re-wet amount is further improved, the basis weight of the water-absorbent resin particles may be, for example, 50 to 450 $g/m^2$, 100 to 400 $g/m^2$, 150 to 375 $g/m^2$, 200 to 350 $g/m^2$, or 225 to 325 $g/m^2$. The basis weight of the water-absorbent resin particles is the total mass of the water-absorbent resin particles per unit area of the absorber.

The shape of the absorber according to the present embodiment may be, for example, a sheet shape. The thickness of the absorber (for example, the thickness of the sheet-shaped absorber) may be 0.1 to 20 mm, or 0.3 to 15 mm.

The absorber 50 (or the absorber 51) can be produced by, for example, a method including laminating the first water absorption layer, the second water absorption layer, and other layers as necessary. Each of the first water absorption layer and the second water absorption layer can be produced by a method including surrounding the water-absorbent resin particles with a sheet-shaped shape retaining member and, as necessary, pressurizing the water-absorbent resin particles.

The absorber described above is, for example, used in various absorbent articles. Examples of the absorbent articles include sanitary materials, agricultural and horticultural materials such as water retention agents and soil conditioners, and industrial materials such as water sealants and anti-condensation agents. Examples of the sanitary materials include diapers (for example, paper diapers), toilet training pants, incontinence pads, sanitary materials (sanitary napkins, tampons, and the like), sweat pads, pet sheets, portal toilet members, and animal excrement treatment materials. The absorbent article may be disposable.

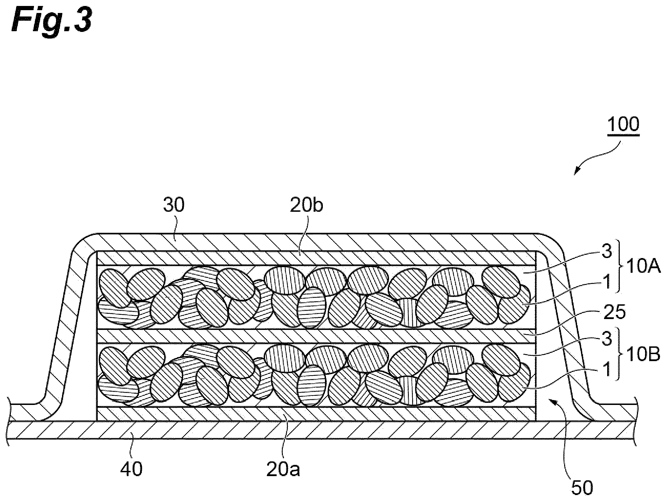

Fig. 3 is a cross-sectional view showing an example of the absorbent article. An absorbent article 100 shown in Fig. 3 includes an absorber 50, a liquid permeable sheet 30, and a liquid impermeable sheet 40. In the absorbent article 100, the liquid impermeable sheet 40, the absorber 50, and the liquid permeable sheet 30 are laminated in this order. The specific aspects of the absorber 50 shown in Fig. 3 may be as described above, but are not limited to a case where the water absorption layer 10A is the first water absorption layer and the water absorption layer 10B is the second water absorption layer, and the water absorption layer 10A may be the second water absorption layer and the water absorption layer 10B may be the first water absorption layer.

The liquid permeable sheet 30 may be a sheet formed of a resin or a fiber usually used in the technical field. The liquid permeable sheet 30 may be a non-woven fabric, a porous sheet, or a combination thereof. The non-woven fabric is a sheet in which fibers are entwined instead of being woven. The non-woven fabric may be a non-woven fabric (short-fiber non-woven fabric) formed of short fibers (that is, staples), or may be a non-woven fabric (long-fiber non-woven fabric) formed of long fibers (that is, filaments). In general, the staples are not limited thereto and may have a fiber length of typically several hundred millimeters or less.

From the viewpoint of liquid permeability, flexibility, and strength in a case where the liquid permeable sheet 30 is used in the absorbent article, the liquid permeable sheet 30 may contain, for example, polyolefin such as polyethylene (PE) or polypropylene (PP), polyester such as polyethylene terephthalate (PET), polytrimethylene terephthalate (PTT), or polyethylene naphthalate (PEN), polyamide such as nylon, synthetic resins such as rayon, regenerated fibers such as cupra, synthetic fibers including acetate or these synthetic resins, or may be natural fibers including cotton, silk, hemp, or pulp (cellulose). The liquid permeable sheet 30 may contain synthetic fibers from the viewpoint of increasing strength of the liquid permeable sheet 30. In particular, the synthetic fibers may be polyolefin fibers, polyester fibers, or a combination thereof. These materials may be used alone, or in combination of two or more kinds thereof.

The liquid permeable sheet 30 may be thermal bonded non-woven fabrics, air-through non-woven fabrics, resin bonded non-woven fabrics, spunbond non-woven fabrics, melt-blown non-woven fabrics, airlaid non-woven fabrics, spunlace non-woven fabrics, point-bonded non-woven fabrics, or a laminate of two or more of non-woven fabrics selected from these non-woven fabrics. These non-woven fabrics can be, for example, non-woven fabrics formed of the above-mentioned synthetic fibers or natural fibers. The laminate of two or more kinds of non-woven fabrics may be, for example, a spunbond/melt-blown/spunbond non-woven fabric that is a composite non-woven fabric having a spunbond non-woven fabric, a melt-blown non-woven fabric, and a spunbond non-woven fabric, which are laminated in this order. A thermal bonded non-woven fabric, an air-through non-woven fabric, a spunbond non-woven fabric, or a spunbond/melt-blown/-spunbond non-woven fabric may be used from the viewpoint of suppressing liquid leakage. In the spunbond/melt-blown/spunbond non-woven fabric, two or more layers of the melt-blown non-woven fabrics may be provided between two layers of the spunbond non-woven fabrics.

It is desirable that the non-woven fabric used as the liquid permeable sheet 30 has appropriate hydrophilicity from the viewpoint of liquid absorption performance of the absorbent article. From this viewpoint, the liquid permeable sheet 30 may be a non-woven fabric having a hydrophilicity of 5 to 200 measured according to a measurement method of Pulp and Paper Test Method No. 68 (2000) by the Japan Technical Association of Pulp and Paper Industry. The hydrophilicity of the non-woven fabric may be 10 to 150. For details of Pulp and Paper Test Method No. 68, WO2011/086843 can be referred to, for example.

The hydrophilic non-woven fabric as described above may be formed of fibers, such as rayon fibers, showing appropriate hydrophilicity, or may be formed of fibers obtained by hydrophilizing hydrophobic chemical fibers such as polyolefin fibers and polyester fibers. Examples of a method of obtaining a non-woven fabric containing hydrophobic chemical fibers that have been hydrophilized include a method of obtaining a non-woven fabric by a spunbond technique using one obtained by mixing a hydrophilizing agent with hydrophobic chemical fibers, a method of using a hydrophilizing agent in a case of preparing a spunbond non-woven fabric with hydrophobic chemical fibers, and a method of impregnating a spunbond non-woven fabric obtained using hydrophobic chemical fibers with a hydrophilizing agent. Examples of the hydrophilizing agent include anionic surfactants such as aliphatic sulfonic acid salts and higher alcohol sulfuric acid ester salts, cationic surfactants such as quaternary ammonium salts, nonionic surfactants such as polyethylene glycol fatty acid esters, polyglycerin fatty acid esters, and sorbitan fatty acid esters, silicone-based surfactants such as polyoxyalkylene-modified silicone, and stain release agents formed of a polyester-based, polyamide-based, acrylic, or urethane-based resin.

From the viewpoint of imparting good liquid permeability, flexibility, strength, and the cushioning property to the absorbent article and from the viewpoint of accelerating the liquid absorption rate of the absorbent article, the liquid permeable sheet 30 may be a non-woven fabric that is moderately bulky and has a large basis weight. The basis weight of the non-woven fabric used in the liquid permeable sheet 30 may be 5 to 200 g/m$^2$, 8 to 150 g/m$^2$, or 10 to 100 g/m$^2$. The thickness of the non-woven fabric used in the liquid permeable sheet 30 may be 20 to 1,400 $\mu$m, 50 to 1,200 $\mu$m, or 80 to 1,000 $\mu$m.

The liquid permeable sheet 30 may consist of two or more sheets, or the surface thereof may be subjected to embossing or perforation in order to improve the diffusibility of the liquid. The embossing or perforation can be performed by a known method. A skin lotion, a moisturizing agent, an antioxidant, an anti-inflammatory agent, a pH adjuster, or the like may be blended into the liquid permeable sheet 30 to reduce irritation to the skin. The shape of the liquid permeable sheet 30 depends on the shapes of the absorber and the absorbent article, but may be a shape that covers the absorber 50 (or the absorber 51) so that the liquid does not leak.

The liquid impermeable sheet 40 is disposed at the outermost portion of the absorbent article 100 on the opposite side from the liquid permeable sheet 30. The liquid impermeable sheet 40 is disposed on a lower side of the shape retaining member 20a in a state of being in contact with the shape retaining member 20a. The liquid impermeable sheet 40 has, for example, a main surface that is larger than the main surface of the absorber 50 (or the absorber 51), and an outer edge of the liquid impermeable sheet 40 extends around the absorber 50 (or the absorber 51). The liquid impermeable sheet 40 prevents the liquid absorbed by the absorber 50 (or the absorber 51) from leaking to the outside from the liquid impermeable sheet 40 side.

Examples of the liquid impermeable sheet 40 include sheets formed of synthetic resins such as polyethylene, polypropylene, and polyvinyl chloride, sheets formed of non-woven fabrics such as spunbond/melt-blown/spunbond (SMS) non-woven fabrics in which a water-resistant melt-blown non-woven fabric is sandwiched between high-strength spunbond non-woven fabrics, and sheets formed of a composite material of these synthetic resins and non-woven fabrics (for example, spunbond non-woven fabrics and spunlace non-woven fabrics). The liquid impermeable sheet 40 may have breathability from the viewpoint that dampness during wearing is reduced, which makes it possible to reduce discomfort to a wearer. As the liquid impermeable sheet 40, a sheet made of a synthetic resin mainly made of a low-density polyethylene (LDPE) resin can be used. For example, the liquid impermeable sheet 40 may be a sheet formed of a synthetic resin and having a basis weight of 10 to 50 g/m$^2$ from the viewpoint of ensuring flexibility such that a sense of wearing the absorbent article is not impaired. In order to impart breathability to the liquid impermeable sheet 40, for example, a filler may be blended into a resin sheet, or the liquid impermeable sheet 40 may be subjected to embossing. As the filler, calcium carbonate or the like is used.

Each member constituting the absorbent article 100 may be adhered. For example, the liquid is more smoothly guided to the absorber by allowing the absorber 50 (or the absorber 51) and the liquid permeable sheet 30 to adhere to each other, and an absorbent article having an improved property of preventing leakage is easily obtained. Examples of the adhering method include known methods of using an adhesive, a heat seal, an ultrasonic seal, and the like.

The absorbent article 100 can be produced, for example, by a method including disposing the absorber 50 between the liquid permeable sheet 30 and the liquid impermeable sheet 40. The laminate obtained by laminating the liquid impermeable sheet 40, the absorber 50, and the liquid permeable sheet 30 in this order is pressurized as necessary.

Fig. 3 is an example, and the configuration of the absorbent article is not limited thereto. The shape of the absorbent article is appropriately determined according to the applications thereof. For example, in a case where the absorbent article is a urine pad or a sanitary napkin, the absorbent article may have a substantially rectangular shape, an elliptical shape, an hourglass shape, or a shuttlecock shape.

The absorbent article may include other members in addition to the liquid permeable sheet, the absorber, and the liquid impermeable sheet described above, as appropriate according to the applications or the functions thereof.

**Examples**

Hereinafter, the present invention will be described in more detail with reference to examples. However, the present invention is not limited to these examples.

[Preparation of Water-Absorbent Resin Particles]

(Production Example 1)

A round-bottomed cylindrical separable flask having an inner diameter of 11 cm and a volume of 2 L and equipped with a reflux cooling device, a dropping funnel, a nitrogen gas introduction tube, and a stirrer (stirrer blade having two stages of four inclined paddle blades with a blade diameter of 5 cm) was prepared. 293 g of n-heptane (hydrocarbon dispersion medium) and 0.736 g of a maleic anhydride-modified ethylene/propylene copolymer (Hi-Wax 1105A, Mitsui Chemicals, Inc., polymeric dispersant) were added to this separable flask to obtain a mixture. The dispersant was dissolved by heating the mixture to 80°C while stirring the mixture at a rotation speed of 300 rpm. Thereafter, the mixture was cooled to 55°C.

Subsequently, 92.0 g of an 80.5 mass% acrylic acid aqueous solution (acrylic acid: 1.03 mol) was put into a triangular flask having a volume of 500 mL. Subsequently, while the mixture was cooled from the outside, 75% by mole of acrylic acid was neutralized by adding 147.7 g of a 20.9 mass% sodium hydroxide aqueous solution dropwise to the mixture. Subsequently, 0.092 g of hydroxyethyl cellulose (HEC AW-15F, SUMITOMO SEIKA CHEMICALS CO., LTD., thickener), 0.0736 g (0.272 mmol) of potassium persulfate (water-soluble radical polymerization initiator), and 0.0101 g (0.0580 mmol) of ethylene glycol diglycidyl ether (internal crosslinking agent) were added and then dissolved, thereby preparing a first stage monomer aqueous solution.

After addition of the above-mentioned first stage monomer aqueous solution to the above-mentioned separable flask, the mixture was stirred for 10 minutes. In addition, a surfactant solution was obtained by heat-dissolving 0.736 g of sucrose stearate (Ryoto Sugar Ester S-370, manufactured by Mitsubishi-Chemical Foods Corporation, surfactant, HLB: 3) in 6.62 g of n-heptane. A reaction solution was obtained by adding 7.356 g of the obtained surfactant solution to the separable flask. Next, the inside of the separable flask system was sufficiently substituted with nitrogen while the reaction solution was stirred at a rotation speed of 400 rpm. Thereafter, the separable flask was immersed in a water bath at 70°C to heat the reaction solution and first stage polymerization was performed for 10 minutes to obtain a first stage reaction mixture.

Subsequently, 128.8 g (acrylic acid: 1.44 mol) of an 80.5 mass% acrylic acid aqueous solution was put into another triangular flask having a volume of 500 mL. Subsequently, while the mixture was cooled from the outside, 75% by mole of acrylic acid was neutralized by adding 159.0 g of a 27 mass% sodium hydroxide aqueous solution dropwise to the mixture. Thereafter, 0.1030 g (0.3810 mmol) of potassium persulfate and 0.0116 g (0.0666 mmol) of ethylene glycol diglycidyl ether (internal crosslinking agent) were added thereto and then dissolved, thereby preparing a second stage monomer aqueous solution.

After the first stage reaction mixture was cooled to 25°C while being stirred at a rotation speed of 1000 rpm, the total amount of the second stage monomer aqueous solution was added to the first stage reaction mixture to obtain a reaction solution. Next, the inside of the system was sufficiently substituted with nitrogen while the reaction solution was stirred. Thereafter, the separable flask was immersed in a water bath at 70°C to heat the reaction solution and second stage polymerization was performed for 5 minutes to obtain a second stage reaction mixture (polymer particles before surface crosslinking).

After the second stage polymerization, the second stage reaction mixture was heated in an oil bath at 125°C and 255 g of water was extracted to the outside of the system by azeotropic distillation of n-heptane and water while the n-heptane was refluxed. Subsequently, 0.0884 g (0.5075 mmol) of ethylene glycol diglycidyl ether was added as a surface crosslinking agent and then the result was held at 83°C for 2 hours to obtain a dispersion liquid of polymer particles after surface crosslinking.

Thereafter, the dispersion liquid of the polymer particles after the above-mentioned surface crosslinking was heated in an oil bath at 125°C, and n-heptane was evaporated and dried, thereby obtaining polymer particles (dried product). The polymer particles were allowed to pass through a sieve having an opening of 850 $\mu$m, thereby obtaining 223.4 g of water-absorbent resin particles (1) in a form in which spherical particles were aggregated.

(Production Example 2)

0.1% by mass of amorphous silica (TOKUSIL NP-S, Oriental Silicas Corporation, hydrophilic) with respect to the mass of the water-absorbent resin particles (1) was mixed with the water-absorbent resin particles (1), thereby obtaining water-absorbent resin particles (2). The water-absorbent resin particles (2) correspond to the water-absorbent resin particles A.

(Production Example 3)

Production Example 1 was repeated, and the collected water-absorbent resin particles (1) were classified with a sieve having an opening of 250 $\mu$m, thereby obtaining 500 g or more of water-absorbent resin particles (3) having a particle diameter of 250 to 850 $\mu$m.

(Production Example 4)

225.1 g of the water-absorbent resin particles (4) in a state of not being coated with the spherical particles in an aggregated form was obtained in the same manner as in Production Example 2 except that the amount of the water to be extracted to the outside of the system by azeotropic distillation was changed to 237 g. The water-absorbent resin particles (4) correspond to the water-absorbent resin particles J.

(Production Example 5)

227.2 g of the water-absorbent resin particles (5) were obtained in the same manner as in Production Example 2 except that 0.092 g (0.339 mmol) of 2,2'-azobis(2-amidinopropane) dihydrochloride as the azo-based compound was added to the first stage monomer aqueous solution, the addition amount of potassium persulfate was changed to 0.028 g (0.102 mmol), the addition amount of ethylene glycol diglycidyl ether was changed to 0.0046 g (0.026 mmol), 0.129 g (0.475 mmol) of 2,2'-azobis(2-amidinopropane) dihydrochloride as the azo-based compound was added to the second stage monomer aqueous solution, the addition amount of potassium persulfate was changed to 0.039 g (0.143 mmol), the amount of water to be extracted to the outside of the system by azeotropic distillation was changed to 234.2 g, the ethylene glycol diglycidyl ether as the surface crosslinking agent was not added, and the mixing amount of the amorphous silica was changed to 0.2% by mass. The water-absorbent resin particles (5) correspond to the water-absorbent resin particles K.

(Production Example 6)

500 g or more of water-absorbent resin particles (6) having a particle diameter of 250 to 850 μm was obtained in the same manner as in Production Example 5 except that the amount of water extracted to the outside of the system by azeotropic distillation was changed to 226.5 g, 0.0884 g (0.5075 mmol) of ethylene glycol diglycidyl ether as a surface crosslinking agent was added, and amorphous silica was not added to the polymer particles (dried product), and the above-mentioned operation was repeated, and the collected water-absorbent resin particles were classified using a sieve with an opening of 250 μm.

(Production Example 7)

The water-absorbent resin particles collected from a commercially available diaper (trade name: Pampers Diapers Fresh NB size, manufactured by The Procter & Gamble Company) sold in China were classified with a sieve having an opening of 250 μm, thereby obtaining 500 g or more of water-absorbent resin particles (7) having a particle diameter of 250 to 850 μm.

(Production Example 8)

A round-bottomed cylindrical separable flask having an inner diameter of 11 cm and a volume of 2 L and equipped with a reflux cooling device, a dropping funnel, a nitrogen gas introduction tube, and a stirrer (stirrer blade having two stages of four inclined paddle blades with a blade diameter of 5 cm) was prepared. 293 g of n-heptane (hydrocarbon dispersion medium) and 0.736 g of a maleic anhydride-modified ethylene/propylene copolymer (Hi-Wax 1105A, Mitsui Chemicals, Inc., polymeric dispersant) were added to this separable flask to obtain a mixture. The dispersant was dissolved by heating the mixture to 80°C while stirring the mixture at a rotation speed of 300 rpm. Thereafter, the mixture was cooled to 55°C.

Subsequently, 92.0 g of an 80.5 mass% acrylic acid aqueous solution (acrylic acid: 1.03 mol) was put into a triangular flask having a volume of 500 mL. Subsequently, while the mixture was cooled from the outside, 75% by mole of acrylic acid was neutralized by adding 147.7 g of a 20.9 mass% sodium hydroxide aqueous solution dropwise to the mixture. Thereafter, 1.38 g of hydroxyethyl cellulose (HEC AW-15F, SUMITOMO SEIKA CHEMICALS CO., LTD., thickener), 0.0736 g (0.272 mmol) of potassium persulfate (water-soluble radical polymerization initiator), and 0.0101 g (0.0580 mmol) of ethylene glycol diglycidyl ether (internal crosslinking agent) were added thereto, and the mixture was dissolved to prepare a monomer aqueous solution.

The above-mentioned monomer aqueous solution was added to the above-mentioned separable flask, and the mixture was stirred for 10 minutes. In addition, a surfactant solution was obtained by heat-dissolving 0.736 g of sucrose stearic acid ester (Ryoto Sugar Ester S-370, manufactured by Mitsubishi-Chemical Foods Corporation, surfactant, HLB: 3) in 6.62 g of n-heptane. A reaction solution was obtained by adding 7.356 g of the obtained surfactant solution to the separable flask. Next, the inside of the separable flask system was sufficiently substituted with nitrogen while the reaction solution was stirred at a rotation speed of 400 rpm. Thereafter, the separable flask was immersed in a water bath at 70°C to heat the reaction solution and polymerization was performed for 10 minutes to obtain a reaction mixture (polymer particles before surface crosslinking). Next, the reaction mixture was heated in an oil bath at 125°C, and 118 g of water was extracted to the outside of the system by azeotropic distillation of n-heptane and water while n-heptane was refluxed, the flask was lifted up in a state where the lower portion thereof was slightly in contact with the oil bath, and the internal temperature was adjusted to 83°C. Subsequently, 0.0368 g (0.213 mmol) of ethylene glycol diglycidyl ether was added as a surface crosslinking agent and then the result was held at an internal temperature of 83°C for 2 hours to obtain a dispersion liquid of polymer particles after surface crosslinking.

Thereafter, the dispersion liquid of the polymer particles after the above-mentioned surface crosslinking was heated in an oil bath at 125°C, and n-heptane was evaporated and dried, thereby obtaining polymer particles (dried product). The polymer particles were allowed to pass through a sieve having an opening of 850 μm, and 0.5% by mass of amorphous silica (TOKUSIL NP-S, Oriental Silicas Corporation, hydrophilic) with respect to the mass of the polymer particles was mixed with the polymer particles, thereby obtaining 91.6 g of spherical water-absorbent resin particles (8). The water-absorbent resin particles (8) correspond to the water-absorbent resin particles I.

(Production Example 9)

The water-absorbent resin particles were collected from a commercially available diaper (trade name: CARE FEEL Lightweight and Refreshing, thin pants type, L Size, manufactured by CAINZ CO., LTD.) sold in Japan, thereby obtaining water-absorbent resin particles (9). The water-absorbent resin particles (9) correspond to the water-absorbent resin particles L.

(Production Example 10)

[Step of Preparing Emulsion Material]

Water and ice were added to a plastic vat with a length of 27 cm, a width of 38 cm, and a depth of 7 cm to prepare an ice bath at 3°C. A glass beaker having an internal volume of 1 L was placed in this ice bath and 897.41 g of ion-exchanged water was added thereto. The ice bath was placed on top of a magnetic stirrer, a stirrer tip was added to the inside of the beaker, and the mixture was stirred.

10.55 g of sodium hydroxide (Granule, NACALAI TESQUE, INC.) was added to a beaker little by little to prepare a sodium hydroxide aqueous solution.

A round-bottomed cylindrical separable flask having an inner diameter of 11 cm and an internal volume of 2 L and equipped with a reflux cooling device, a thermometer, and a stirrer (stirrer blades having four inclined paddle blades having a blade diameter of 5 cm) was prepared. 100 g of an ethylene-acrylic acid copolymer (SK Global Chemical: Primacor 5980i) was added to this flask. Thereafter, the total amount of the above-mentioned sodium hydroxide aqueous solution was added thereto. Thereafter, the beaker used to prepare the sodium hydroxide aqueous solution was washed with 50.0 g of ion-exchanged water and the washing water was added to the separable flask to obtain a reaction solution.

While the reaction solution was stirred with a stirrer at a rotation speed of 500 rpm, the result was immersed in an oil bath at 103°C and the internal temperature was raised to 95°C. Thereafter, the temperature of the oil bath was appropriately adjusted such that the internal temperature was 95°C to 97°C, which was maintained for 4 hours.

Thereafter, the separable flask was pulled up from the oil bath and left to be naturally cooled at room temperature until the internal temperature reached 35°C. After confirming that the internal temperature was 35°C or lower, the reaction solution was filtered through a nylon mesh having an opening of 108 μm and a 10% water-dispersed emulsion material of an ethylene-sodium acrylate copolymer having a neutralization degree of 95% was obtained as the filtrate.

[Coating Step]

500.0 g of a 10% water-dispersed emulsion material of an ethylene-sodium acrylate copolymer having a neutralization degree of 95% and 5.0 g of polyethylene glycol (PEG6000, Tokyo Chemical Industry Co., Ltd.) were mixed in a polybeaker having an internal volume of 1 L, thereby preparing a coating material (1).

500.0 g of water-absorbent resin particles (3) were injected into the container of a fluidized bed granulator and hot air at 50°C was blown from the bottom of the container. Subsequently, while 505 g of the coating material (1) was dried, the coating material (1) was sprayed on the water-absorbent resin particles whirled up by blowing. After spraying the coating material, drying was carried out at 50°C for 30 minutes. After the drying, coated-resin particles were obtained.

50.0 g of the obtained coated-resin particles were spread in a metal vat 26 cm long and 20 cm wide and the vat was covered with aluminum foil. The aluminum foil was perforated, and the coated-resin particles were heated for 60 minutes by a hot air dryer (ADVANTEC, FV-320) set at 100°C, thereby obtaining 50.0 g of water-absorbent resin particles B having a coating layer.

(Production Example 11)

In the coating step, 150.0 g of a 25% water-dispersed emulsion (ZAIKTHENE N, SUMITOMO SEIKA CHEMICALS CO., LTD.) of an ethylene-sodium acrylate copolymer as an emulsion material and 3.75 g of polyethylene glycol were mixed, and 221.25 g of ion-exchanged water was added thereto for dilution, thereby preparing a coating material (2). 50.0 g of water-absorbent resin particles C having a coating layer were obtained in the same manner as in Production Example 10 except that the coating material (2) was used instead of the coating material (1), the addition amount of the coating material was changed to 375 g, and the set temperature of the hot air dryer was changed to 80°C.

(Production Example 12)

In the coating step, 200.0 g of a 25% water-dispersed emulsion (ZAIKTHENE N, SUMITOMO SEIKA CHEMICALS CO., LTD.) of an ethylene-sodium acrylate copolymer as an emulsion material and 5.0 g of polyethylene glycol were mixed, and 295.0 g of ion-exchanged water was added thereto for dilution, thereby preparing a coating material (3). 50.0 g of water-absorbent resin particles D having a coating layer were obtained in the same manner as in Production Example 11 except that the coating material (3) was used instead of the coating material (2), the addition amount of the coating material was changed to 500 g, and the heating time of the hot air dryer was changed to 30 minutes.

(Production Example 13)

In the coating step, 1000.0 g of a 10% water-dispersed emulsion material of an ethylene-sodium acrylate copolymer having a neutralization degree of 95% as an emulsion material and 25.0 g of polyethylene glycol (PEG6000, Tokyo Chemical Industry Co., Ltd.) were mixed, thereby preparing a coating material (4). 50.0 g of water-absorbent resin particles E having a coating layer were obtained in the same manner as in Production Example 10 except that the coating material (4) was used instead of the coating material (1), the addition amount of the coating material was changed to 1025 g, and the set temperature of the hot air dryer was changed to 90°C.

(Production Example 14)

In the coating step, 1500.0 g of a 10% water-dispersed emulsion material of an ethylene-sodium acrylate copolymer having a neutralization degree of 95% as an emulsion material and 25.0 g of polyethylene glycol (PEG6000, Tokyo Chemical Industry Co., Ltd.) were mixed, thereby preparing a coating material (5). 50.0 g of water-absorbent resin particles F having a coating layer were obtained in the same manner as in Production Example 13 except that the coating material (5) was used instead of the coating material (4) and the addition amount of the coating material was changed to 1525 g.

(Production Example 15)

In the coating step, 50.0 g of water-absorbent resin particles G having a coating layer were obtained in the same manner as in Production Example 13 except that the water-absorbent resin particles (6) were used instead of the water-absorbent resin particles (3), the set temperature of the hot air dryer was changed to 95°C, and the heating time of the hot air dryer was changed to 30 minutes.

(Production Example 16)

In the coating step, 50.0 g of water-absorbent resin particles H having a coating layer were obtained in the same manner as in Production Example 10 except that the water-absorbent resin particles (7) were used instead of the water-absorbent resin particles (3) and the heating time of the hot air dryer was changed to 30 minutes.

[Evaluation of water-absorbent resin particles]

The water-absorbent resin particles were evaluated as follows.

(1) Water retention capacity

The water retention capacity (room temperature) of physiological saline of the water-absorbent resin particles was measured by the following procedure. First, a cotton bag (cotton broadcloth No. 60, 100 mm in width $\times$ 200 mm in length) into which 2.0 g of the weighed water-absorbent resin particles were placed in a beaker having the internal volume of 500 mL. After pouring 500 g of physiological saline into the cotton bag containing the water-absorbent resin particles at one time so that lumps could not be produced, the upper portion of the cotton bag was bound with a rubber band and the cotton bag was left to stand for 30 minutes to swell the water-absorbent resin particles. The cotton bag after an elapse of 30 minutes was dehydrated for 1 minute using a dehydrator (product number: H-122, manufactured by KOKUSAN CO., LTD.) which had been set to have the centrifugal force of 167 G, and the mass Wa [g] of the cotton bag containing the swollen gel after dehydration was measured. The same operation was performed without addition of the water-absorbent resin particles, the empty mass Wb [g] at the time in a case where the cotton bag was wet was measured, and the water retention capacity of the physiological saline of the water-absorbent resin particles was calculated from the following equation.

$$\text{Water retention capacity [g/g]} = (Wa - Wb)/2.0$$

(2) One-Minute Value of Lockup Height

The one-minute value of the lockup height was measured by the following method. 0.200 g of particles for evaluation was precisely weighed and arranged in a layered shape on the bottom of an acrylic cylinder having an inner diameter of 2.0 cm and a depth of 8.0 cm to form a particle layer with a flat upper surface, and a height H0 of the particle layer was then measured. Thereafter, 20 g of physiological saline at 25°C was poured at once from the upper portion of the acrylic cylinder. The measurement was started from the time point at which the total amount of physiological saline was put in, the height H1 of the particle layer after 1 minute was read, and the one-minute value of the lockup height was calculated from the following equation.

Further, in a case where the upper surface of the particle layer after the water absorption was not flat, the height of the highest portion was defined as H1.

$$\text{One-minute value (cm) of lockup height} = H1 - H0$$

(3) Water Absorption Rate according to Voltex Method

A 100 mL beaker having a rotor (8 mm × 30 mm, no ring) was charged with 50.0 g of physiological saline and maintained at 25°C in a constant-temperature tank. Subsequently, 2.0 g of the water-absorbent resin particles for evaluation were injected into a vortex of the physiological saline stirred at 600 rpm to start measurement with a stopwatch at the same time. The time point when the vortex disappeared and the liquid level became horizontal was defined as an end point, and the time (seconds) until then was defined as the water absorption rate.

[Preparation of Absorber]

(Production Example A)

A total of 6.0 g of the water-absorbent resin particles (one or a plurality of kinds of the water-absorbent resin particles A to L, the same applies hereinafter) and 4.0 g of crushed pulp (Rayfloc, manufactured by Rayonier Inc.) were used in the proportions listed in Table 2 and uniformly mixed by air papermaking (suction pressure of 0.5 to 0.6 MPa, conveyor speed of 5.3 sec/cm) with an air flow type mixer (Padformer, manufactured by O-tec Co., Ltd.), thereby preparing an absorber core with a size of 40 cm × 12 cm. Subsequently, in a state of sandwiching the upper portion and the lower portion of the absorber core between two sheets of tissue paper having the same area as that of the absorber core and having a basis weight of 16 g/m$^2$, a load of 141 kPa was applied to the entirety for 30 seconds to press it, thereby preparing a water absorption layer A.

A water absorption layer B containing the water-absorbent resin particles in the proportion listed in Table 2 was prepared in the same manner as that for the water absorption layer A. An absorber was prepared by laminating the water absorption layers A and B from above such that four sides of the outer peripheries of the water absorption layers A and B overlapped with each other. The absorber was prepared by vertically laminating the water absorption layers such that four sides of the outer peripheries of the water absorption layers overlapped with each other. Further, an air-through type polyethylene porous liquid permeable sheet having a basis weight of 22 g/m$^2$ and having the same area as that of the absorber was disposed on the upper surface of the absorber to obtain an absorbent article.

(Production Example B)

An airlaid non-woven fabric (KNH Enterprise Co., Ltd.) having a basis weight of 40 g/m$^2$ was cut into two pieces with a size of 14 cm × 42 cm, and each of the pieces was defined as an airlaid non-woven fabric 1 and an airlaid non-woven fabric 2.

A total amount of 0.2 g of a hot melt adhesive (Henkel Japan Ltd., ME-765E) was applied to the airlaid non-woven fabric 2 in 14 straight lines at intervals of 10 mm with a hot melt coating machine (HALLYS Corporation, pump: Marshal 150, table: XA-DT, tank set temperature: 150°C, set temperature in hose: 165°C, gun head set temperature: 170°C). The application pattern of the adhesive was a spiral stripe. During coating, four sides of the outer peripheral sides (width 1 cm) of the airlaid non-woven fabric 2 were covered with a cover so that the adhesive was not applied thereto.

7.2 g of the water-absorbent resin particles were uniformly sprayed, in a range of 12 cm × 40 cm, to the central portion of the surface of the airlaid non-woven fabric 2 to which the hot melt had adhered, thereby preparing a water absorption layer D.

0.2 g of the hot melt was applied to the airlaid non-woven fabric 1 by the same operation as mentioned above but without covering the outer periphery thereof. 7.2 g of the water-absorbent resin particles in the proportion listed in Table 4 were uniformly sprayed, in a range of 12 cm × 40 cm, to the central portion of the surface of the airlaid non-woven fabric 1 to which the hot melt had adhered, thereby preparing a water absorption layer C.

An air-through non-woven fabric (Guangzhou Junqian Nonwoven Co., Ltd., basis weight of 45 g/m$^2$) with a size of 40 cm × 12 cm was placed on the surface of the airlaid non-woven fabric 2 onto which the water-absorbent resin particles had been sprayed, and both ends of the air-through non-woven fabric 1 and the airlaid non-woven fabric 2 were aligned such that the adhesive-coated surface was positioned on the inside with peeling paper sandwiched therebetween, and the air-through non-woven fabric 1 and the airlaid non-woven fabric 2 were bonded to each other by being pressed under conditions of 110°C and 0.1 MPa using a laminating machine (model: HP-600LFS, manufactured by Hashima Co., Ltd., Straight Linear Fusing Press), thereby preparing an absorbent. The range in which the water-absorbent resin particles were sprayed in the absorber was 12 cm × 40 cm. An air-through type polyethylene porous liquid permeable sheet having a basis weight of 22 g/m$^2$ and having the same area as that of the absorber was further disposed on the upper surface of the absorber, thereby obtaining an absorbent article. In the absorbent article, the air-through type polyethylene porous liquid permeable sheet, the water absorption layer C, the air-through non-woven fabric, and the water absorption layer D were disposed in this order.

(Production Example C)

A total of 7.2 g of the water-absorbent resin particles (one or a plurality of kinds of the water-absorbent resin particles A to L, the same applies hereinafter) and 4.8 g of crushed pulp (Rayfloc, manufactured by Rayonier Inc.) were used in the proportions listed in Table 5 and uniformly mixed by air papermaking (suction pressure of 0.5 to 0.6 MPa, conveyor speed of 5.3 sec/cm) with an air flow type mixer (manufactured by O-tec Co., Ltd., Padformer), thereby preparing an absorber core with a size of 40 cm × 12 cm. Subsequently, in a state of sandwiching the upper portion and the lower portion of the absorber core between two sheets of tissue paper having the same area as that of the absorber core and having a basis weight of 16 g/m$^2$, a load of 141 kPa was applied to the entirety for 30 seconds to press it, thereby preparing a water absorption layer E.

An airlaid non-woven fabric (KNH Enterprise Co., Ltd.) having a basis weight of 40 g/m$^2$ was cut into two pieces with a size of 14 cm × 42 cm, and each of the pieces was defined as an airlaid non-woven fabric 1 and an airlaid non-woven fabric 2.

A total amount of 0.2 g of a hot melt adhesive (Henkel Japan Ltd., ME-765E) was applied to the airlaid non-woven fabric 2 in 14 straight lines at intervals of 10 mm with a hot melt coating machine (HALLYS Corporation, pump: Marshal 150, table: XA-DT, tank set temperature: 150°C, set temperature in hose: 165°C, gun head set temperature: 170°C). The application pattern of the adhesive was a spiral stripe. During coating, four sides of the outer peripheral sides (width 1 cm) of the airlaid non-woven fabric 2 were covered with a cover so that the adhesive was not applied thereto.

7.2 g of the water-absorbent resin particles were uniformly sprayed, in a range of 12 cm × 40 cm, to the central portion of the surface of the airlaid non-woven fabric 2 to which the hot melt had adhered.

0.2 g of the hot melt was applied to the airlaid non-woven fabric 1 by the same operation as mentioned above but without covering the outer periphery thereof. Both ends of the airlaid non-woven fabric 2 and the airlaid non-woven fabric 1 were aligned such that the adhesive-coated surfaces were on the inner side, and with peeling paper sandwiched therebetween, pressing was performed under the conditions of 110°C and 0.1 MPa to attach using a laminating machine (model: HP-600LFS, manufactured by Hashima Co., Ltd., Straight Linear Fusing Press), thereby preparing a water absorption layer F.

An absorber was prepared by superimposing the region of the water absorption layer E where the water-absorbent resin particles were sprayed and the region of the water absorption layer F where the water-absorbent resin particles were sprayed to coincide with each other. An air-through type polyethylene porous liquid permeable sheet having a basis weight of 22 g/m$^2$ and having the same area as that of the absorber was further disposed on the upper surface of the absorber, thereby obtaining an absorbent article. In the absorbent article, the air-through type polyethylene porous liquid permeable sheet, the water absorption layer E, the air-through non-woven fabric, and the water absorption layer F were disposed in this order from above.

(Production Example D)

An absorbent article was prepared by disposing the air-through type polyethylene porous liquid permeable sheet, the water absorption layer F, the air-through non-woven fabric, and the water absorption layer E in this order from above using the water absorption layer E and the water absorption layer F prepared in Production Example C.

(Production Example E)

A total of 4.0 g of the water-absorbent resin particles (one or a plurality of kinds of the water-absorbent resin particles A to L, the same applies hereinafter) and 2.7 g of crushed pulp (Rayfloc, manufactured by Rayonier Inc.) were used in proportions listed in Table 7 and uniformly mixed by air papermaking (suction pressure of 0.5 to 0.6 MPa, conveyor speed of 5.3 sec/cm) with an air flow type mixer (manufactured by O-tec Co., Ltd., Padformer), thereby preparing an absorber core with a size of 40 cm × 12 cm. Subsequently, in a state of sandwiching the upper portion and the lower portion of the absorber core between two sheets of tissue paper having the same area as that of the absorber core and having a basis weight of 16 g/m$^2$, a load of 141 kPa was applied to the entirety for 30 seconds to press it, thereby preparing a water absorption layer G.

A water absorption layer H and a water absorption layer I containing the water-absorbent resin particles in the proportions listed in Table 7 were prepared in the same manner as that for the water absorption layer G. An absorber was prepared by laminating the water absorption layer G, the water absorption layer H, and the water absorption layer I from above such that four sides of the outer peripheries of the water absorption layers overlapped with each other. Further, an air-through type polyethylene porous liquid permeable sheet having a basis weight of 22 g/m$^2$ and having the same area as that of the absorber was disposed on the upper surface of the absorber to obtain an absorbent article.

(Example 1)

An absorbent article was prepared by the production method of Production Example A using 4.8 g of the water-absorbent resin particles A and 1.2 g of the water-absorbent resin particles B as the water-absorbent resin particles of the water absorption layer A and the water absorption layer B.

(Example 2)

An absorbent article was prepared by the production method of Production Example A using 4.8 g of the water-absorbent resin particles A and 1.2 g of the water-absorbent resin particles B as the water-absorbent resin particles of the water absorption layer A and 6.0 g of the water-absorbent resin particles A as the water-absorbent resin particles of the water absorption layer B.

(Example 3)

An absorbent article was prepared by the production method of Production Example A using 6.0 g of the water-absorbent resin particles A as the water-absorbent resin particles of the water absorption layer A and 4.8 g of the water-absorbent resin particles A and 1.2 g of the water-absorbent resin particles B as the water-absorbent resin particles of the water absorption layer B.

(Example 4)

An absorbent article was prepared by the production method of Production Example A using 5.4 g of the water-absorbent resin particles A and 0.6 g of the water-absorbent resin particles B as the water-absorbent resin particles of the water absorption layer A and 6.0 g of the water-absorbent resin particles A as the water-absorbent resin particles of the water absorption layer B.

(Example 5)

An absorbent article was prepared by the production method of Production Example A using 3.6 g of the water-absorbent resin particles A and 2.4 g of the water-absorbent resin particles B as the water-absorbent resin particles of the water absorption layer A and 6.0 g of the water-absorbent resin particles A as the water-absorbent resin particles of the water absorption layer B.

(Example 6)

An absorbent article was prepared by the production method of Production Example A using 1.2 g of the water-absorbent resin particles A and 4.8 g of the water-absorbent resin particles B as the water-absorbent resin particles of the water absorption layer A and 6.0 g of the water-absorbent resin particles A as the water-absorbent resin particles of the water absorption layer B.

(Example 7)

An absorbent article was prepared by the production method of Production Example A using 4.8 g of the water-absorbent resin particles A and 1.2 g of the water-absorbent resin particles C as the water-absorbent resin particles of the water absorption layer A and 6.0 g of the water-absorbent resin particles A as the water-absorbent resin particles of the water absorption layer B.

(Example 8)

An absorbent article was prepared by the production method of Production Example A using 4.8 g of the water-absorbent resin particles A and 1.2 g of the water-absorbent resin particles D as the water-absorbent resin particles of the water absorption layer A and 6.0 g of the water-absorbent resin particles A as the water-absorbent resin particles of the water absorption layer B.

(Example 9)

An absorbent article was prepared by the production method of Production Example A using 4.8 g of the water-absorbent resin particles A and 1.2 g of the water-absorbent resin particles E as the water-absorbent resin particles of the water absorption layer A and 6.0 g of the water-absorbent resin particles A as the water-absorbent resin particles of the water absorption layer B.

(Example 10)

An absorbent article was prepared by the production method of Production Example A using 4.8 g of the water-absorbent resin particles A and 1.2 g of the water-absorbent resin particles G as the water-absorbent resin particles of the water absorption layer A and 6.0 g of the water-absorbent resin particles A as the water-absorbent resin particles of the water absorption layer B.

(Example 11)

An absorbent article was prepared by the production method of Production Example A using 4.8 g of the water-absorbent resin particles A and 1.2 g of the water-absorbent resin particles H as the water-absorbent resin particles of the water absorption layer A and 6.0 g of the water-absorbent resin particles A as the water-absorbent resin particles of the water absorption layer B.

(Example 12)

An absorbent article was prepared by the production method of Production Example A using 4.8 g of the water-absorbent resin particles A and 1.2 g of the water-absorbent resin particles I as the water-absorbent resin particles of the water absorption layer A and 6.0 g of the water-absorbent resin particles A as the water-absorbent resin particles of the water absorption layer B.

(Comparative Example 1)

An absorbent article was prepared by the production method of Production Example A using 6.0 g of the water-absorbent resin particles A as the water-absorbent resin particles of the water absorption layer A and the water absorption layer B.

(Comparative Example 2)

An absorbent article was prepared by the production method of Production Example A using 4.8 g of the water-absorbent resin particles A and 1.2 g of the water-absorbent resin particles J as the water-absorbent resin particles of the water absorption layer A and 6.0 g of the water-absorbent resin particles A as the water-absorbent resin particles of the water absorption layer B.

(Comparative Example 3)

An absorbent article was prepared by the production method of Production Example A using 4.8 g of the water-absorbent resin particles A and 1.2 g of the water-absorbent resin particles K as the water-absorbent resin particles of the water absorption layer A and 6.0 g of the water-absorbent resin particles A as the water-absorbent resin particles of the water absorption layer B.

(Example 13)

An absorbent article was prepared by the production method of Production Example A using 4.8 g of the water-absorbent resin particles L and 1.2 g of the water-absorbent resin particles B as the water-absorbent resin particles of the water absorption layer A and 6.0 g of the water-absorbent resin particles A as the water-absorbent resin particles of the water absorption layer B.

(Example 14)

An absorbent article was prepared by the production method of Production Example A except that 1.92 g of the water-absorbent resin particles A and 0.48 g of the water-absorbent resin particles B were used as the water-absorbent resin particles of the water absorption layer A, the amount of crushed pulp of the water absorption layer A was changed to 1.6 g, 9.6 g of the water-absorbent resin particles A were used as the water-absorbent resin particles of the water absorption layer B, and the amount of crushed pulp of the water absorption layer B was changed to 6.4 g.

(Comparative Example 4)

An absorbent article was prepared by the same method as in Example 14 except that the water-absorbent resin particles of the water absorption layer A was changed to 2.4 g of the water-absorbent resin particles A.

(Example 15)

An absorbent article was prepared by the production method of Production Example A except that 7.68 g of the water-absorbent resin particles A and 1.92 g of the water-absorbent resin particles B were used as the water-absorbent resin particles of the water absorption layer A, the amount of crushed pulp of the water absorption layer A was changed to 6.4 g, 2.4 g of the water-absorbent resin particles A were used as the water-absorbent resin particles of the water absorption layer B, and the amount of crushed pulp of the water absorption layer B was changed to 1.6 g.

(Comparative Example 5)

An absorbent article was prepared by the same method as in Example 15 except that the water-absorbent resin particles of the water absorption layer A was changed to 9.6 g of the water-absorbent resin particles A.

(Example 16)

An absorbent article was prepared by the same method as in Example 2 except that the amount of crushed pulp of the water absorption layer A was changed to 14.0 g and the amount of crushed pulp of the water absorption layer B was changed to 14.0 g.

(Comparative Example 6)

An absorbent article was prepared by the same method as in Example 16 except that the water-absorbent resin particles of the water absorption layer A was changed to 6.0 g of the water-absorbent resin particles A.

(Example 17)

An absorbent article was prepared by the production method of Production Example B using 4.8 g of the water-absorbent resin particles A and 2.4 g of the water-absorbent resin particles B as the water-absorbent resin particles of the water absorption layer C and 7.2 g of the water-absorbent resin particles A as the water-absorbent resin particles of the water absorption layer D.

(Example 18)

An absorbent article was prepared by the production method of Production Example B using 4.8 g of the water-absorbent resin particles A and 2.4 g of the water-absorbent resin particles D as the water-absorbent resin particles of the water absorption layer C and 7.2 g of the water-absorbent resin particles A as the water-absorbent resin particles of the water absorption layer D.

(Example 19)

An absorbent article was prepared by the production method of Production Example B using 4.8 g of the water-absorbent resin particles A and 2.4 g of the water-absorbent resin particles F as the water-absorbent resin particles of the water absorption layer C and 7.2 g of the water-absorbent resin particles A as the water-absorbent resin particles of the water absorption layer D.

(Comparative Example 7)

An absorbent article was prepared by the production method of Production Example B using 7.2 g of the water-absorbent resin particles A as the water-absorbent resin particles of the water absorption layer C and 7.2 g of the water-absorbent resin particles A as the water-absorbent resin particles of the water absorption layer D.

(Example 20)

An absorbent article was prepared by the production method of Production Example C using 5.76 g of the water-absorbent resin particles A and 1.44 g of the water-absorbent resin particles B as the water-absorbent resin particles of the water absorption layer E and 7.2 g of the water-absorbent resin particles A as the water-absorbent resin particles of the water absorption layer F.

(Comparative Example 8)

An absorbent article was prepared by the production method of Production Example C using 7.2 g of the water-absorbent resin particles A as the water-absorbent resin particles of the water absorption layer E and 7.2 g of the water-absorbent resin particles A as the water-absorbent resin particles of the water absorption layer F.

(Example 21)

An absorbent article was prepared by the production method of Production Example D using 5.76 g of the water-absorbent resin particles A and 1.44 g of the water-absorbent resin particles B as the water-absorbent resin particles of the water absorption layer E and 7.2 g of the water-absorbent resin particles A as the water-absorbent resin particles of the water absorption layer F.

(Comparative Example 9)

An absorbent article was prepared by the production method of Production Example D using 7.2 g of the water-absorbent resin particles A as the water-absorbent resin particles of the water absorption layer E and 7.2 g of the water-absorbent resin particles A as the water-absorbent resin particles of the water absorption layer F.

(Example 22)

An absorbent article was prepared by the production method of Production Example E using 2.8 g of the water-absorbent resin particles A and 1.2 g of the water-absorbent resin particles B as the water-absorbent resin particles of the water absorption layer G, 4.0 g of the water-absorbent resin particles A as the water-absorbent resin particles of the water absorption layer H, and 4.0 g of the water-absorbent resin particles A as the water-absorbent resin particles of the water absorption layer I.

(Comparative Example 10)

An absorbent article was prepared by the production method of Production Example E using 4.0 g of the water-absorbent resin particles A as the water-absorbent resin particles of the water absorption layer G, 4.0 g of the water-absorbent resin particles A as the water-absorbent resin particles of the water absorption layer H, and 4.0 g of the water-absorbent resin particles A as the water-absorbent resin particles of the water absorption layer I.

[Overlapping Region of Water Absorption Layers]

The ratio (S1/S2 × 100) of an area S1 of a region 1 where one water absorption layer was provided and overlapped with a region 2 to an area S2 of the region 2 where the other water absorption layer was provided in plan view was 100%. The term "one water absorption layer" is the water absorption layer B in Production Example A, the water absorption layer D in Production Example B, the water absorption layer F in Production Examples C and D, and the water absorption layer H or the water absorption layer I in Production Example E. The term "the other water absorption layer" is the water absorption layer A in Production Example A, the water absorption layer C in Production Example B, the water absorption layer E in Production Examples C and D, and the water absorption layer G in Production Example E.

[Basis Weight of Water-Absorbent Resin Particles]

The basis weight of the water-absorbent resin particles was 250 g/m² in the absorber obtained by the method of Production Example A, and the basis weight of the water-absorbent resin particles was 300 g/m² in the absorbers obtained by the methods of Production Examples B, C, D, and E.

[Preparation of Test Solution]

9866.0 g of distilled water, 100.0 g of sodium chloride, 3.0 g of calcium chloride dihydrate, 6.0 g of magnesium chloride hexahydrate, 25.0 g of a 1% by mass Triton X solution (mixture of Triton X-100, manufactured by FUJIFILM Wako Pure Chemical Corporation and water), and 0.25 g of food blue No. 1 (for coloration) were mixed to prepare a test solution.

[Absorption Rate]

The test was performed in an indoor environment adjusted to a temperature of 25°C and a humidity of 50% (RH). The absorbent article was placed on a horizontal table. A liquid injection cylinder (59.80 g) having an opening with an outer diameter of 3.5 cm and an inner diameter of 3 cm was placed on the central portion of the absorbent article, and 80 mL of the test solution was injected into the cylinder at one time. The time from the start of injection to the completion of absorption was measured as the absorption rate. Immediately after the absorption was completed, the cylinder was removed from the absorbent article.
Next, 30 minutes after the start of injection of the test solution, the addition of artificial urine was performed for the second time in the same manner as the first time, and the absorption rate was measured. The operations were further repeated three times, and the total number of times of the operation was five times. The absorption rate was obtained by adding up the absorption rates during the injection of the test solution five times.

(Re-wet Amount)

Immediately after the completion of water absorption of the test solution of the injection for the fifth time, the cylinder was removed, and 1 hour after the injection of the test solution, about 75 g of filter paper (ADVANTEC No.51A, formed to 100 mm × 100 mm) whose mass had been preliminarily measured was placed near the center of the absorbent article, about 0.7 psi of a weight (bottom surface: 100 mm × 100 mm, 5.0 kg) was quickly placed thereon, and a load was applied thereto for 5 minutes. Thereafter, the weight and the filter paper were removed, and the mass of the test solution absorbed by the filter paper was measured. The increase amount of the filter paper before and after the test was defined as the re-wet amount (g).

[Table 1]

| Production Example | Water-absorbent resin particles | Water retention capacity [g/g] | One-minute value of lockup height [cm] | Water absorption rate [sec] |
|---|---|---|---|---|
| 2 | A | 42 | 1.7 | 36 |
| 10 | B | 40 | 0.2 | 297 |
| 11 | C | 39 | 0.4 | 114 |
| 12 | D | 40 | 0.2 | 101 |
| 13 | E | 34 | 0.2 | 742 |
| 14 | F | 32 | 0.2 | 451 |
| 15 | G | 44 | 0.1 | 273 |
| 16 | H | 38 | 0.2 | 282 |
| 8 | I | 39 | 1.0 | 131 |
| 4 | J | 31 | 2.7 | 44 |
| 5 | K | 84 | 1.5 | 74 |

(continued)

| Production Example | Water-absorbent resin particles | Water retention capacity [g/g] | One-minute value of lockup height [cm] | Water absorption rate [sec] |
|---|---|---|---|---|
| 9 | L | 42 | 2.0 | 33 |

[Table 2]

Production Example A

| | Water absorption layer A | | | | | | Water absorption layer B | | | | | | Water absorption rate [sec] | Re-wet amount [g] |
|---|---|---|---|---|---|---|---|---|---|---|---|---|---|---|
| | Type | Used amount [g] | Water-absorbent resin particles Type | Water-absorbent resin particles Used amount [g] | Total amount [g] | Amount of crushed pulp [8] | Type | Used amount [g] | Water-absorbent resin particles Type | Water-absorbent resin particles Used amount [g] | Total amount [g] | Amount of crushed pulp [8] | | |
| Example 1 | A | 4.8 | B | 1.2 | 6.0 | 4.0 | A | 4.8 | B | 1.2 | 6.0 | 4.0 | 266 | 56 |
| Example 2 | A | 4.8 | B | 1.2 | 6.0 | 4.0 | A | 6.0 | - | - | 6.0 | 4.0 | 279 | 60 |
| Example 3 | A | 6.0 | - | - | 6.0 | 4.0 | A | 4.8 | B | 1.2 | 6.0 | 4.0 | 267 | 57 |
| Example 4 | A | 5.4 | B | 0.6 | 6.0 | 4.0 | A | 6.0 | - | - | 6.0 | 4.0 | 265 | 62 |
| Example 5 | A | 3.6 | B | 2.4 | 6.0 | 4.0 | A | 6.0 | - | - | 6.0 | 4.0 | 265 | 59 |
| Example 6 | A | 1.2 | B | 4.8 | 6.0 | 4.0 | A | 6.0 | - | - | 6.0 | 4.0 | 244 | 63 |
| Example 7 | A | 4.8 | C | 1.2 | 6.0 | 4.0 | A | 6.0 | - | - | 6.0 | 4.0 | 265 | 63 |
| Example 8 | A | 4.8 | D | 1.2 | 6.0 | 4.0 | A | 6.0 | - | - | 6.0 | 4.0 | 254 | 64 |
| Example 9 | A | 4.8 | E | 1.2 | 6.0 | 4.0 | A | 6.0 | - | - | 6.0 | 4.0 | 246 | 62 |
| Example 10 | A | 4.8 | G | 1.2 | 6.0 | 4.0 | A | 6.0 | - | - | 6.0 | 4.0 | 247 | 56 |
| Example 11 | A | 4.8 | H | 1.2 | 6.0 | 4.0 | A | 6.0 | - | - | 6.0 | 4.0 | 242 | 55 |
| Example 12 | A | 4.8 | I | 1.2 | 6.0 | 4.0 | A | 6.0 | - | - | 6.0 | 4.0 | 236 | 61 |
| Comparative Example 1 | A | 6.0 | - | - | 6.0 | 4.0 | A | 6.0 | - | - | 6.0 | 4.0 | 297 | 60 |
| Comparative Example 2 | A | 4.8 | J | 1.2 | 6.0 | 4.0 | A | 6.0 | - | - | 6.0 | 4.0 | 259 | 66 |
| Comparative Example 3 | A | 4.8 | K | 1.2 | 6.0 | 4.0 | A | 6.0 | - | - | 6.0 | 4.0 | 332 | 54 |
| Example 13 | L | 4.8 | B | 1.2 | 6.0 | 4.0 | A | 6.0 | - | - | 6.0 | 4.0 | 222 | 60 |

[Table 3]

| | Production Example A | | | | | | | | | | | | |
| | Water absorption layer A | | | | | Water absorption layer B | | | | | | Water absorption rate [sec] | Re-wet amount [g] |
| | Water-absorbent resin particles | | | | Amount of crushed pulp [g] | Water-absorbent resin particles | | | | Amount of crushed pulp [g] | | |
| | Type | Used amount [g] | Type | Used amount [g] | Total amount [g] | | Type | Used amount [g] | Type | Used amount [g] | Total amount [g] | | | |
| Example 14 | A | 1.92 | B | 0.48 | 2.4 | 1.6 | A | 9.6 | - | - | 9.6 | 6.4 | 275 | 59 |
| Comparative Example 4 | A | 2.4 | - | - | 2.4 | 1.6 | A | 9.6 | - | - | 9.6 | 6.4 | 279 | 62 |
| Example 15 | A | 7.68 | B | 1.92 | 9.6 | 6.4 | A | 2.4 | - | - | 2.4 | 1.6 | 238 | 59 |
| Comparative Example 5 | A | 9.6 | - | - | 9.6 | 6.4 | A | 2.4 | - | - | 2.4 | 1.6 | 249 | 61 |
| Example 16 | A | 4.8 | B | 1.2 | 6.0 | 14.0 | A | 6.0 | - | - | 6.0 | 14.0 | 102 | 66 |
| Comparative Example 6 | A | 6.0 | - | - | 6.0 | 14.0 | A | 6.0 | - | - | 6.0 | 14.0 | 106 | 68 |

EP 4 509 106 A1

[Table 4]

| | Production Example B | | | | | | | Water absorption rate [sec] | Re-wet amount [g] |
|---|---|---|---|---|---|---|---|---|---|
| | Water absorption layer C | | | | | Water absorption layer D | | | |
| | Water-absorbent resin particles | | | | | Water-absorbent resin particles | | | |
| | Type | Used amount [g] | Type | Used amount [g] | Total amount [g] | Type | Used amount [g] | | |
| Example 17 | A | 4.8 | B | 2.4 | 7.2 | A | 7.2 | 143 | 33 |
| Example 18 | A | 4.8 | D | 2.4 | 7.2 | A | 7.2 | 151 | 32 |
| Example 19 | A | 4.8 | F | 2.4 | 7.2 | A | 7.2 | 123 | 34 |
| Comparative Example 7 | A | 7.2 | - | - | 7.2 | A | 7.2 | 153 | 34 |

[Table 5]

| | Production Example C | | | | | | | | Water absorption rate [sec] | Re-wet amount [g] |
|---|---|---|---|---|---|---|---|---|---|---|
| | Water absorption layer E | | | | | | Water absorption layer F | | | |
| | Water-absorbent resin particles | | | | | Amount of crushed pulp [g] | Water-absorbent resin particles | | | |
| | Type | Used amount [g] | Type | Used amount [g] | Total amount [g] | | Type | Used amount [g] | | |
| Example 20 | A | 5.76 | B | 1.44 | 7.2 | 4.8 | A | 7.2 | 291 | 41 |
| Comparative Example 8 | A | 7.2 | - | - | 7.2 | 4.8 | A | 7.2 | 299 | 42 |

[Table 6]

| | Production Example D | | | | | | | | Water absorption rate [sec] | Re-wet amount [g] |
|---|---|---|---|---|---|---|---|---|---|---|
| | Water absorption layer E | | | | | | Water absorption layer F | | | |
| | Water-absorbent resin particles | | | | | Amount of crushed pulp [8] | Water-absorbent resin particles | | | |
| | Type | Used amount [g] | Type | Used amount [g] | Total amount [g] | | Type | Used amount [g] | | |
| Example 21 | A | 5.76 | B | 1.44 | 7.2 | 4.8 | A | 7.2 | 157 | 47 |
| Comparative Example 9 | A | 7.2 | - | - | 7.2 | 4.8 | A | 7.2 | 163 | 47 |

[Table 7]

| | Production Example E | | | | | | | | | | |
| | Water absorption layer G | | | | | Amount of crushed pulp [8] | Water absorption layers H and I | | Amount of crushed pulp [8] | Water absorption rate [sec] | Re-wet amount [g] |
| | Water-absorbent resin particles | | | | | | Water-absorbent resin particles | | | | |
| | Type | Used amount [g] | Type | Used amount [g] | Total amount [8] | | Type | Used amount [8] | | | |
| Example 22 | A | 2.8 | B | 1.2 | 4.0 | 2.7 | A | 4.0 | 2.7 | 249 | 58 |
| Comparative Example 10 | A | 4.0 | - | - | - | 2.7 | A | 4.0 | 2.7 | 270 | 58 |

28

EP 4 509 106 A1

The evaluation results are listed in Tables 2 to 7. The absorbers of the examples including the water absorption layer containing the water-absorbent resin particles in which the water retention capacity of the physiological saline and the one-minute value of the lockup height were adjusted to predetermined values were multilayer type absorbers, and both a high permeation rate and a small re-wet amount were achieved.

Reference Signs List

1: water-absorbent resin particle
3: fibrous material
10A, 10B, 10C: water absorption layer
20a, 20b: shape retaining member
25, 25a, 25b: separator
30: liquid permeable sheet
40: liquid impermeable sheet
50, 51: absorber
100: absorbent article

**Claims**

1. An absorber comprising:

   a first water absorption layer; and
   a second water absorption layer laminated on the first water absorption layer,
   wherein the first water absorption layer contains water-absorbent resin particles (I) having a water retention capacity of physiological saline of 32 to 80 g/g and a one-minute value of a lockup height of 1.6 cm or less,
   the second water absorption layer contains water-absorbent resin particles (II) having a one-minute value of a lockup height of more than 1.6 cm, and
   the one-minute value of the lockup height is measured by the following procedures,

      (1) 0.200 g of water-absorbent resin particles are disposed over an entire bottom surface in a cylinder having an inner diameter of 2.0 cm and a depth of 8.0 cm to form a particle layer, and a height of the particle layer is denoted by H0 [cm],
      (2) 20 g of physiological saline is injected to the particle layer to swell the particle layer, and
      (3) the height of the particle layer is recorded as H1' one minute after a total amount of the physiological saline is injected, and a one-minute value [cm] of the lockup height is calculated from Expression: H1' - H0.

2. The absorber according to claim 1,

   wherein a content of the water-absorbent resin particles (I) contained in the first water absorption layer is in a range of 5% to 95% by mass with respect to a total amount of water-absorbent resin particles contained in the first water absorption layer, and
   a content of the water-absorbent resin particles (II) contained in the second water absorption layer is in a range of 60% to 100% by mass with respect to a total amount of water-absorbent resin particles contained in the second water absorption layer.

3. The absorber according to claim 1 or 2,
   wherein a basis weight of water-absorbent resin particles is in a range of 50 to 450 g/m$^2$.

4. The absorber according to claim 1 or 2,
   wherein at least one of the first water absorption layer or the second water absorption layer contains a fibrous material.

5. The absorber according to claim 1 or 2,
   wherein the absorber includes a plurality of layers of at least one of the first water absorption layer or the second water absorption layer.

6. The absorber according to claim 1 or 2,
   wherein a water absorption rate of the water-absorbent resin particles (I) according to a Vortex method is more than 150 seconds.

7. The absorber according to claim 1 or 2,
   wherein the water-absorbent resin particles (I) are coated-resin particles having a coating layer covering at least a part of a surface.

8. An absorbent article comprising:
   the absorber according to claim 1 or 2.

# *Fig.1*

50

20b
3 ⎤
  ⎬ 10A
1 ⎦
25
3 ⎤
  ⎬ 10B
1 ⎦
20a

*Fig.2*

# Fig.3

## INTERNATIONAL SEARCH REPORT

International application No.

**PCT/JP2023/017275**

### A. CLASSIFICATION OF SUBJECT MATTER

*A61F 13/53*(2006.01)i; *A61F 13/534*(2006.01)i; *A61F 13/537*(2006.01)i
FI: A61F13/534 100; A61F13/53 300; A61F13/537 210

According to International Patent Classification (IPC) or to both national classification and IPC

### B. FIELDS SEARCHED

Minimum documentation searched (classification system followed by classification symbols)

A61F13/53; A61F13/534; A61F13/537

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2021/075508 A1 (SUMITOMO SEIKA CHEMICALS CO., LTD.) 22 April 2021 (2021-04-22) paragraphs [0007]-[0198], fig. 1-12 | 1-4, 8 |
| Y | | 5-7 |
| Y | WO 2021/039715 A1 (SUMITOMO SEIKA CHEMICALS CO., LTD.) 04 March 2021 (2021-03-04) paragraphs [0006]-[0202], fig. 1-6 | 5 |
| Y | WO 2021/117780 A1 (SUMITOMO SEIKA CHEMICALS CO., LTD.) 17 June 2021 (2021-06-17) paragraphs [0015]-[0016], [0027]-[0034] | 6-7 |
| A | JP 2021-122734 A (NIPPON CATALYTIC CHEM IND) 30 August 2021 (2021-08-30) paragraphs [0013]-[0179], fig. 1-10 | 1-8 |

☐ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **28 June 2023** | **11 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2023/017275**

| Patent document cited in search report | | | Publication date (day/month/year) | Patent family member(s) | | | Publication date (day/month/year) |
|---|---|---|---|---|---|---|---|
| WO | 2021/075508 | A1 | 22 April 2021 | (Family: none) | | | |
| WO | 2021/039715 | A1 | 04 March 2021 | CN | 114269307 | A | |
| | | | | KR 10-2022-0050918 | | A | |
| WO | 2021/117780 | A1 | 17 June 2021 | US | 2023/0036813 | A1 | |
| | | | | paragraphs [0020]-[0021], [0032]-[0039] | | | |
| | | | | EP | 4074755 | A1 | |
| | | | | KR 10-2022-0117238 | | A | |
| | | | | CN | 114787245 | A | |
| JP | 2021-122734 | A | 30 August 2021 | CN | 113244054 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019141309 A

- WO 2011086843 A